(19) **Europäisches Patentamt**
**European Patent Office**
**Office européen des brevets**

(11) **EP 4 765 863 A1**

(12) **EUROPEAN PATENT APPLICATION**

(43) Date of publication:
**24.06.2026   Bulletin 2026/26**

(21) Application number: **24222802.1**

(22) Date of filing: **23.12.2024**

(51) International Patent Classification (IPC):
**H04R 1/10** (2026.01)          **A61F 11/06** (2006.01)
**G10K 11/16** (2006.01)        **G10L 21/00** (2013.01)
**H04R 3/00** (2006.01)

(52) Cooperative Patent Classification (CPC):
**H04R 1/1016; A61F 11/06; A61F 11/145;**
**G10K 11/16; H04R 1/1041; H04R 1/1083;**
**H04R 3/005;** G10K 2210/1081; G10L 21/0208;
H04R 2201/107; H04R 2460/01; H04R 2460/13

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **INVISIO A/S**
**2650 Hvidovre (DK)**

(72) Inventors:
• **TOFT, Tobias**
**2650 Hvidovre (DK)**
• **WAGNER, Mikkel**
**2650 Hvidovre (DK)**
• **BODÉN, Richard**
**2650 Hvidovre (DK)**
• **PEDERSEN, John Oldenburg**
**2650 Hvidovre (DK)**

(74) Representative: **Guardian**
**IP Consulting I/S**
**Diplomvej, Building 381**
**2800 Kgs. Lyngby (DK)**

(54)    **IN-EAR TACTICAL COMMUNICATION AND/OR HEARING PROTECTION SYSTEM**

(57)    The present invention relates to a communication system configured to be used in a demanding environment, the communication system comprising at least one communication and hearing protection device (103, 303a, 303b), in particular at least one in-ear communication and hearing protection device (103, 303a, 303b), configured to be worn by a user (101), the communication and hearing protection device (103, 303a, 303b) comprising an ambient microphone (315, 315a, 315b) configured to provide an ambient microphone input signal (505a, 505b, 513), and a transmit (Tx) microphone (317, 317a, 317b) configured to provide a transmit microphone input signal (503a, 503b, 511) in response to registering speech from the user (101), one or more processors (205, 207) configured to implement and execute a trained artificial intelligence or machine learning method or component (515, 607) to generate a correction function or signal (517, 609) in response to the ambient microphone input signal (505a, 505b, 513) and the transmit microphone input signal (503a, 503b, 511).

FIG. 5

EP 4 765 863 A1

## Description

### TECHNICAL FIELD

The present invention relates in one aspect to an in-ear tactical hearing protection and/or communication system to be used in a demanding and high-noise environment where a trained artificial intelligence or machine learning method or component is implemented and executed. The invention relates according to another aspect, to method of training an artificial intelligence or machine learning method or component to be executed by a device of a communication system.

### BACKGROUND

Hearing loss is by far the most prevalent service-connected disability among veteran soldiers worldwide. Where sensorineural hearing loss, caused by damage to the inner ear and auditory nerve, result in permanent irreversible loss of hearing for the individual resulting in difficulty understanding speech which can cause social isolation and a significant drop in life quality. Conditions such as auditory processing disorder and hearing loss is often associated with blast exposure from gunfire and grenade explosions or other severe noise exposure both short and long-term.

Even though hearing loss may be helped through the use of hearing aids, it is desirable to preserve natural hearing abilities and prevent any external nigh noise exposures for individuals who are required to operate in demanding environments.

Hearing protection devices are generally known and used amount soldiers, police forces, etc. for noise attenuation. Typically, passive hearing protecting devices such as foam earplugs or earmuffs are used to physically block or damp sound waves thereby protecting the user against harmful noise exposure; however this type of hearing protecting devices block out all types of sound, which can be extremely problematic for many types of operations where it can be critical that the user is able to maintain situational awareness of the surroundings. To maintain situational awareness, active hearing protecting devices may be used relying on a combination of passive sound attenuation, speakers, and microphones typically in combination with noise filtration or other for transmitting ambient sound into the ear canal of the user while maintaining the noise level below a predetermined limit.

Additionally, clear and undistorted communication is vital for military and public safety professionals operating in extreme and demanding environments. Clear and undistorted communication is very significant e.g. for soldiers, police, rescue personnel, fire fighters, and other task forces as it ensures or at least facilitates improved coordination among team members and enhances safety and security by reducing misunderstandings. Rapid and swift communication facilitates quick decision-making and response to changing situations. The ability to share observations and intelligence in real-time between relevant groups or individuals relies on clear and undistorted communication and enhances mission success and maximizes safety. Being able to transmit or receive the correct voice communication in a complex communication setup, independent of audio communication device types and under stressful circumstances, can make the difference between life and death.

The ability to receive or transmit field intelligence is essential to coordinate any special operation successfully. Targeted communication between units, individuals, and/or central commands is essential and can make the difference between success or complete failure. The need for communication is high and the complexity of different operations are increasing as multiple different groups or individuals may join forces and work closely together to accomplish a mission. Forces such as police, rescue personal, and firefighters may collaborate at an emergency site or different special forces or marine troopers from different countries or organizations may collaborate in a joint coalition force. Such constellations require complex and often dynamic communication steps, configurations, setups, etc. containing and/or involving multiple communication devices, communication channels etc. making unambiguous and reliable communication flow challenging. Multiple communication channels may be available e.g. with different levels of confidentiality in relation to a message classification level thereby making administration and control of communication links challenging.

Thus, for personal operating in demanding environments with high noise exposure, it is essential to wear tactical hearing protection and communication equipment allowing for both situational awareness, clear communication, and hearing protecting. Additionally, factors such as comfort and ease of use is extremely important for such devices to support the user in the best possible way as the devices are often worn for long durations during for example military operations. Generally, in-ear hearing protection provide better hearing protection and comfort as they fit directly into the ear canal of the user compared to circumaural hearing protection headsets. However, in-ear hearing protection devices are more challenging to fit properly and adapt with microphones for communication purpose as they are situated within the ear canal of the user.

### SUMMARY

One object of the present invention is to overcome at least some of the above-mentioned drawbacks and/or other disadvantages (at least to an extent), or at least to provide an alternative to existing solutions.

According to a first aspect disclosed herein are embodiments of a communication system according to independent claim 1 with advantageous embodiments as defined by the dependent claims and as disclosed herein.

According to the above first aspect, disclosed herein are embodiments of a communication system configured to be used in a demanding environment as disclosed herein.

The communication system comprises at least one communication and hearing protection device, in particular at least one in-ear communication and hearing protection device, configured to be worn by a user. The communication and hearing protection device comprises an ambient microphone configured to provide an ambient microphone input signal and a transmit (Tx) microphone configured to provide a transmit microphone input signal in response to registering speech from the user. The label 'transmit' refer to that, at least in some embodiments, the transmit microphone input signal (or rather a processed form thereof, processed according to the first aspect and embodiments thereof) is intended to be transmitted, e.g. as a communication (Tx) signal via a radio or other communication device).

The communication system further comprises one or more processors configured to implement and execute a trained artificial intelligence or machine learning method or component to generate a correction function or signal in response to the ambient microphone input signal and the transmit microphone input signal. The generated enables a corrected or improved voice signal.

In some embodiments, only a correction function is generated. In some alternative embodiments, only a correction signal is generated.

In some embodiments, the correction function or signal enables a corrected or improved voice signal and/or the one or more processors is/are further configured to apply the correction function or signal to the transmit microphone input signal thereby providing a corrected or improved voice signal. The corrected and/or improved part may relate to both speech optimisation (i.e. a clearer and more understandable voice signal) and noise suppression/removal specifically removing noise contributions from the demanding environment.

In some embodiments, the corrected or improved voice signal is provided as signal for transmission, e.g. to be received by one or more communication devices, e.g. by one or more one or more radios.

In some embodiments, the transmit (Tx) microphone is

- a vibration-based transducer, e.g. an accelerometer type vibration sensitive transducer such as a digital voice pick up (VPU) type microphone, acoustically coupled with the user, when the user is wearing the communication and hearing protection device, where the vibration-based transducer is configured to obtain a voice signal of the user in response to vibrations caused by the user speaking and provide the transmit microphone input signal in response thereto.

In some embodiments, the ambient microphone is

- a microphone configured to register an airborne ambient acoustic signal of a demanding environment and provide the ambient microphone input signal in response thereto.

In some embodiments,

- the ambient microphone input signal represents an airborne acoustic signal, and
- the transmit microphone input signal represents a vibration signal.

In some embodiments, the generated correction function or signal is or comprises data representing a gain vector or similar, wherein the gain vector or similar comprises a number of predicted adjustment values, predicted by the trained artificial intelligence or machine learning method or component, for different segments or parts of the transmit microphone input signal or a processed version thereof, where an adjustment value for a particular segment or part specify whether the transmit microphone input signal or a processed version thereof in the particular segment or part should be kept or should be increased or decreased and to what extent.

In some embodiments, predicted adjustment values each are limited or clipped to be within predetermined maximum and/or minimum threshold, wherein the predetermined maximum and/or minimum threshold are set in response to a derived or estimated sound pressure value representing or estimating an external background noise level, wherein the predetermined maximum and/or minimum threshold are set to relatively high threshold(s) in case the derived or estimated sound pressure value indicates no or little background noise and are set to relatively low threshold(s) in case the derived or estimated sound pressure value indicates a relatively high background noise.

In some further embodiments, the external background noise level is represented measured by the ambient microphone input signal.

In some embodiments, the communication system is configured to:

- apply a processed version of the generated correction function or signal to the transmit microphone input signal thereby filtering or reducing noise from the transmit microphone input signal and providing the corrected or improved voice signal.

In some embodiments, the transmit (Tx) microphone is digital, and wherein the communication system or the communication and hearing protection device comprises a dedicated direct digital-to-analog converter (DAC) circuitry coupled (e.g. or preferably directly) to the transmit (Tx) microphone and configured to perform lossless front-end digital to analog signal conversion. This is advantageous since no additional noise is generated/introduced in this manner and nor will the signal be degraded as a consequence of an additional "signal path" and/or additional logical operation(s) by other circuits and components.

In some embodiments, the transmit (Tx) microphone is configured to output a digital Pulse Density Modulation (PDM) signal representing an obtained voice signal of the user, and wherein the dedicated direct digital-to-analog converter (DAC) circuitry is configured to receive the digital Pulse Density Modulation (PDM) signal and to convert it into an analog signal using a D-FlipFlop and an active lowpass filter, preferably applying a fourth order Bessel function, comprised by the dedicated direct digital-to-analog converter (DAC) circuitry.

In some embodiments, the one or more processors is further configured

- to process the transmit microphone input signal to generate a transmit magnitude component thereof, or
- to process the transmit microphone input signal to generate a further processed transmit magnitude component thereof,

and to provide the generated transmit magnitude component or the generated further processed transmit magnitude component as input to the trained artificial intelligence or machine learning method or component to generate the correction function or signal.

In some embodiments, the one or more processors is further configured

- to process the ambient microphone input signal to generate an ambient magnitude component thereof, or
- to process the ambient microphone input signal to generate a further processed ambient magnitude component thereof,

and to provide the generated ambient magnitude component or the generated further processed ambient magnitude component as input to the trained artificial intelligence or machine learning method or component to generate the correction function or signal.

In some embodiments, the one or more processors is further configured

- to process the generated ambient magnitude component to derive a derived or estimated sound pressure value thereof, the derived or estimated sound pressure value representing or estimating a background noise level,
- to adjust the correction function or signal, prior to the correction function or signal being applied to the transmit microphone input signal, in response to the derived or estimated sound pressure value resulting in an adjusted correction function or signal, and
- applying the adjusted correction function or signal to the transmit microphone input signal instead of the correction function or signal in order to provide the corrected or improved voice signal.

In some further embodiments, the one or more processors is/are further configured

- to, instead of applying the correction function or signal to the transmit microphone input signal, perform noise filtration of the generated further processed transmit magnitude component in response to (or taking into account) the adjusted correction function or signal resulting in a noise corrected further processed transmit magnitude component, and
- providing the corrected or improved voice signal in response to the noise corrected further processed transmit magnitude component.

In some embodiments, the communication system comprises a push-to-talk control unit comprising one or more, e.g. all, of the one or more processors. In some alternative embodiments, the in-ear communication and hearing protection device comprises one or more, e.g. all, of the one or more processors. In some further embodiments, the one or more processors, or their functionality, is shared between the push-to-talk (PTT) control unit and the in-ear communication and hearing protection device, and/or e.g. shared with one or more other devices (of the communication system).

In some embodiments, some or all of the processing functionality of the one or more processors is in another device (than the PTT and the in-ear communication and hearing protection device).

In some embodiments, the communication system comprises one or more of

- a wireless remote PTT device,
- one or more communication devices,
- one or more radios,
- a radio of a first type and a radio of a second type, and
- one or more end-user-devices (EUDs).

In some embodiments, the computer program or routine implementing the trained artificial intelligence or machine learning method or component has been trained according to the training method (701) according to the second aspect as disclosed herein.

In some embodiments, the communication system comprises two communication and hearing protection devices, each corresponding to the communication and hearing protection device as described above, where a first communication and hearing protection device is configured to be inserted in the right ear of the user and the other second communication and hearing protection device is configured to be inserted in the left ear of the user, wherein

the first communication and hearing protection device is configured to

- provide a first ambient microphone input signal by a first ambient microphone,
- a first transmit microphone input signal by a first transmit (Tx) microphone,

the second communication and hearing protection device is configured to

- provide a second ambient microphone input signal by a second ambient microphone,
- a second transmit microphone input signal by a second transmit (Tx) microphone, and

wherein the

- one or more processors is/are configured to implement and execute the trained artificial intelligence or machine learning method or component to generate the correction function or signal in response to a combination of the first and the second ambient microphone input signals and a combination of the first and the second transmit microphone input signal,

wherein the one or more processors is/are configured to apply the correction function or signal to the combined transmit microphone input signal in order to provide the corrected or improved voice signal.

According to a second aspect disclosed herein are embodiments of a method according to independent claim 18 with advantageous embodiments as defined by the dependent claims and as disclosed herein.

According to the above second aspect, disclosed herein are embodiments of a method of training an artificial intelligence or machine learning method or component to be executed by at least one device of a communication system, as disclosed herein.

The artificial intelligence or machine learning method or component is configured to generate real-time processing of a user's voice signal in a demanding environment, where the method comprises

a) obtaining first data representing a reference speech signal including a speech signal of a user obtained in accordance with a first way,
b) obtaining second data representing a training transmit (Tx) signal including the speech signal obtained in accordance with a second way,
c) obtaining third data representing a training ambient signal including the speech signal obtained in accordance with a third way,

d) providing the second data and the third data to the artificial intelligence or machine learning method or component generating a predicted output in response thereto,
e) comparing the predicted output and the first data and determining a difference therebetween, and
f) updating parameters of the artificial intelligence or machine learning method or component in response to the determined difference,

wherein the method further comprises repeating steps a) - f) for new first, second, and third data a plurality of times, typically a large number of times, until the generated difference of the predicted output and the first data is within a predetermined threshold or the improvement of generated difference (from cycle to cycle) stops improving sufficiently.

In some embodiments,

- the first way comprises providing an acoustic signal by a microphone or transducer of a first type being a high quality professional stationary voice recording microphone or transducer,
- the second way comprises providing an acoustic signal by a microphone or transducer of a second type being a vibration pick-up sensor, and/or
- the third way comprises providing an acoustic signal by a microphone or transducer of a third type being an ambient microphone.

In some embodiments,

- the second data, representing a training transmit (Tx) signal including the speech signal, further includes user-generated (such as natural or involuntary) noise e.g. or preferably obtained in accordance with the second way, and/or

- the third data, representing a training ambient signal including the speech signal, further includes user-generated noise e.g. or preferably obtained in accordance with the third way.

In some embodiments,

- the second data, representing a training transmit (Tx) signal including the speech signal, further includes noise being representative of loud noises of a demanding environment,
- the third data, representing a training ambient signal including the speech signal, further includes noise being representative of loud noises of a demanding environment, and
- the first data, representing a reference speech signal including the speech signal, does not include noise being representative of loud noises of a demanding environment.

In some embodiments,

- the noise being representative of loud noises of a demanding environment of the second data has been obtained in accordance with the second way, and/or

- the noise being representative of loud noises of a demanding environment of the third data has been obtained in accordance with the third way.

In some embodiments, the noise being representative of loud noises of a demanding environment of the second data and/or the noise being representative of loud noises of a demanding environment of the third data have been obtained at the same time and/or have been obtained when the user is not speaking.

In some embodiments, the method further comprises

- processing the second data to generate a transmit magnitude component thereof, and providing the generated transmit magnitude component to the artificial intelligence or machine learning method or component instead of providing the second data in step d), and/or

- processing the third data to generate an ambient magnitude component thereof and providing the generated ambient magnitude component to the artificial intelligence or machine learning method or component instead of providing the third data in step d).

## BRIEF DESCRIPTION OF THE DRAWINGS

Embodiments of the invention will now be described in more detail. Various embodiments of the systems and/or the methods according to the different aspects as disclosed herein will be described in connection with the appended drawings, in which:

FIG. 1 schematically illustrates an example of a user wearing an exemplary personal in-ear tactical hearing protection and communication system configured to be worn by a person in communication with other connected device, people or both;

FIG. 2A schematically illustrates an example of a PTT control unit;

FIG. 2B schematically illustrates an example of a PTT-button allocation scheme;

FIG. 2C schematically illustrates an example of a configuration of communication devices connected to a PTT control unit, such as the one of FIG. 2A, and a corresponding PTT button allocation scheme;

FIG. 2D schematically illustrates an example of a modified configuration of communication devices connected to a PTT control unit and a corresponding PTT button allocation scheme;

FIG. 3A schematically illustrates an example of an improved in-ear tactical communication and hearing protection system comprising an in-ear tactical communication and hearing protecting headset connected to a PTT control unit via a cable and a connector;

FIG. 3B schematically illustrates an example of a right (R) earpiece of the in-ear tactical communication and hearing protection headset of FIG. 3A when fitted properly inside the right ear-canal of a user;

FIG. 3C schematically illustrates an example of an exploded view of the right (R) earpiece of the in-ear tactical communication and hearing protection headset of Fig. 3A;

FIG. 3D schematically illustrates an example of a cross-sectional view of the ear tip of the in-ear tactical communication and hearing protection headset of Fig. 3A.

FIG. 3E schematically illustrates an example of a Right R earpiece having an attached ear tip 323 in a compressed state;

FIG. 3F schematically illustrates an example of a Right R earpiece being inserted into the ear canal of a user's right ear having an attached ear tip in a less compressed state;

FIG. 3G schematically illustrates an example of a DAC circuitry located in the earpiece of the in-ear tactical communication and hearing protection headset of FIG. 3A configured to perform a lossless front-end digital to analog signal conversion of a digital output signal from Tx microphone;

FIG. 4A illustrates an example of the standardized test setup used for quantifying the expansion time of the in-ear tactical communication and hearing protecting headset foam tip;

FIG. 4B schematically illustrates a cross-sectional view of a stainless-steel acoustic coupler unit of the test setup of FIG. 4A used for measuring foam-tip expansion time;

FIG. 4C Illustrates a graphical representation of a data series showing the expansion time measurements for the foam-type ear tip according to the present disclosure plotted together with expansion measurements of prior art examples;

FIG. 5 schematically illustrates an example of a processing architecture of the in-ear tactical communication and hearing protecting system configured to remove noise and enhance the speech signal quality of the user;

FIG. 6A schematically illustrates an example of a processing method executed by an in-ear tactical communication and hearing protecting system as disclosed herein to provide a clear and undistorted voice signal via a communication device in demanding environments;

FIG. 6B schematically illustrates an example of a combined pre-processing- and feature extraction step for a Tx microphone input signal according to embodiments of the processing method executed by the in-ear tactical communication and hearing protecting system;

FIG. 6C schematically illustrates an example of a combined pre-processing- and feature extraction step for an ambient microphone input signal according to embodiments of the processing method executed by the in-ear tactical communication and hearing protecting system;

FIG. 6D schematically illustrates an example of a neural network processing step using a deep neural network (DNN) model according to embodimetns of the processing method executed by the in-ear tactical communication and hearing protecting system;

FIG. 6E schematically illustrates an example of an output postprocessing step according to embodiments of the processing method executed by the in-ear tactical communication and hearing protecting system;

FIG. 6F schematically illustrates an example of a noise filtration step according to embodiments of the processing method executed by the in-ear tactical communication and hearing protecting system;

FIG. 6G schematically illustrates an example of a feature reconstruction step according to embodiments of the processing method executed by the in-ear tactical communication and hearing protecting system;

FIG. 7A schematically illustrates an example of a training method applied to train the neural network model of FIG. 6D to provide real-time processing of a user's voice signal to produce clear and undistorted communication in demanding environments;

FIG. 7B schematically illustrates an example of a data collection process used for the training of the neural network model according to some embodiments;

FIG. 7C Illustrates a graphical representation of three exemplary audio signal data series in a first subplot, a second subplot, and a third subplot arranged in a vertical stack, representing training data used to train the training of the neural network model to obtain trained neural network model according to the training method of FIG. 7A; and

FIG. 7D Illustrates a graphical representation of two exemplary audio signal data series in a fourth subplot and a fifth subplot segment arranged in a vertical stack, representing noise data used to train the training of the neural network model to obtain a trained neural network model according to the training method of FIG. 7A.

## DETAILED DESCRIPTION

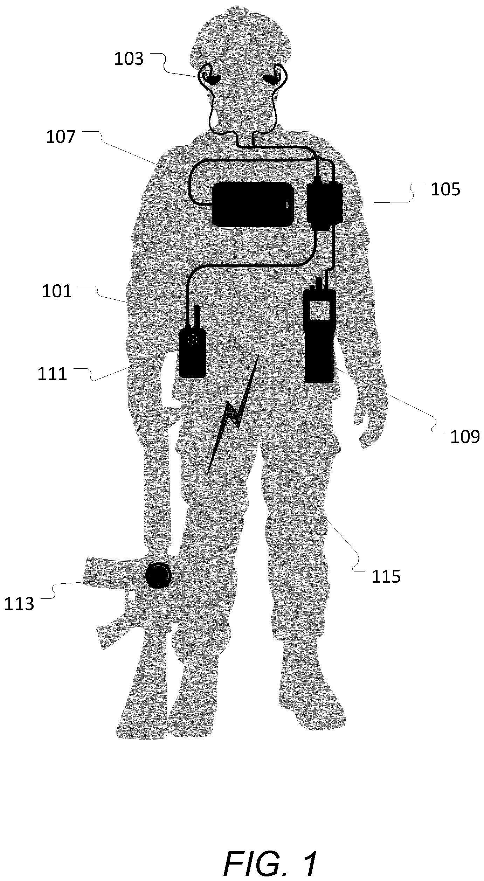

Fig. 1 schematically illustrates an example of a user wearing an exemplary personal in-ear tactical hearing protection and communication system configured to be worn by a person in communication with other connected units (e.g. devices, persons or both).

Illustrated is a user 101 wearing a wearable personal in-ear tactical hearing protection and communication system. In the illustrated example, the personal in-ear tactical hearing protection and communication system comprises one or more devices for tactical communication and an in-ear communication and hearing protection device 103. In the illustrated example, the user 101 is in a so-called dismounted configuration that refers to a situation where a user 101 may maintain one or more communication links with one or more remote parts (system(s) and/or other user(s) via their respective personal communication equipment) (e.g. such as an in-ear tactical hearing protection and communication system) without a physical link (such as a cable, etc). Being dismounted enables the user 101 to freely move around while being able to maintain communication.

The personal in-ear tactical hearing protection and communication system may comprise an in-ear communication and hearing protection device 103, e.g. in the form of a pair of in-ear earbuds configured to be arranged the ear canal of the user 101, and one or more PTT control units 105, 113. Additionally, the user 101 may carry and use one or more additional electronic devices (such as a Tactical Display Unit (TDU) or End-User-Device (EUD) 107) and one or more communication devices 109, 111 operably connected to the PTT control unit 105 and to the headset 103 for establishing audio and data links with other remote units, teams or devices.

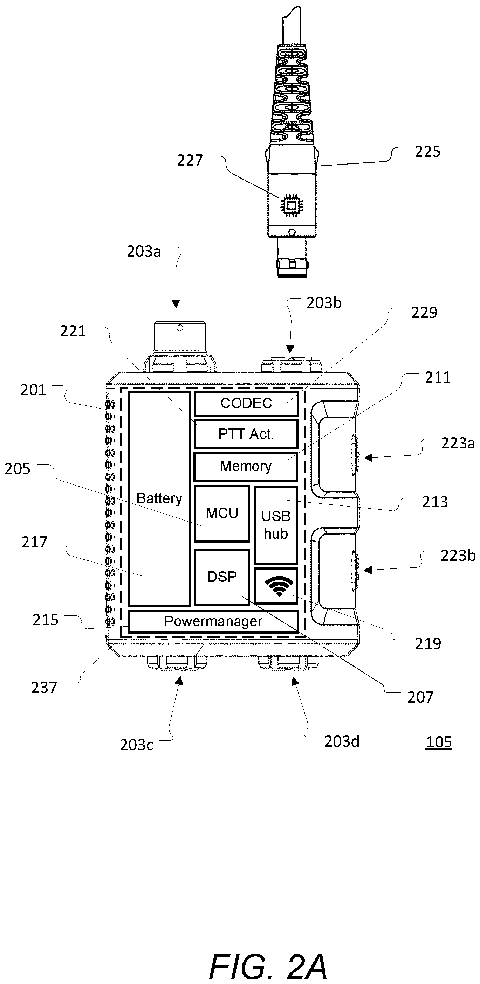

In the illustrated example, the user 101 is wearing an in-ear hearing protection headset 103, a PTT control unit 105 (also sometimes referred to a PTT control hub or box or simply control box), an EUD 107, and two radios 109, 111 of different types or of different settings, where the in-ear headset 103, the EUD 107, and the two radios 109, 111 respectively is connected to the PTT control unit 105 via respective cables. Alternatively, one or more of the cabled connections may be replaced by suitable wireless connection(s). Additionally, a wireless remote PTT device 113 containing additional PTT button(s) may be located elsewhere on the user 101 or equipment for easy operation during action and/or expanding the number of available PTT-buttons. The wireless remote PTT may be in wireless connection 115 with the PTT control unit 105 for transmitting PTT button press actions or other control actions to the main PTT control unit 105. The PTT control unit 105 may e.g. be a PTT control unit as disclosed herein, e.g. as described in connection with Fig. 2A and elsewhere.

## Communication devices

A communication device may be a handheld radio 109, 111 providing voice and data communication e.g. in the VHF and UHF bands and offering secure and reliable communication in various operational environments such as an AN/PRC-152 or another radio 109, 111 for dismounted use providing interoperable communication, e.g. in multiple frequency bands, and supports voice and data transmission.

A communication device may e.g. be a specialised radio system tailored for mounted operations, including the AN/VRC-110, a multiband radio system featuring components like the AN/PRC-117F(C) or AN/PRC-117G(C); the Thales AN/PRC-148 Vehicle Adapter Amplifier (VAA), which adapts the AN/PRC-148 handheld radio for vehicular use; the Collins Aerospace AN/ARC-210, primarily an airborne radio system but also utilized in some mounted vehicle applications for secure voice and data communication; and the Barrett PRC-2091, a vehicular-mounted version of the Barrett PRC-2090 HF tactical radio, offering long-range communication capability in the HF band for mounted operations. These systems are important for providing reliable communication links between military vehicles and command centers and facilitating coordination and situational awareness on the battlefield or in emergency situations. A communication device may be connected to the PTT control unit 105 via a dedicated cable containing a plurality of terminals for exchanging, control signals, power, analog voice signals, and data signals as generally known in the field.

A communication device may e.g. also be a mobile phone, a satellite phone, etc. Generally, connected communication devices can be wired or wirelessly connected to another device/communication device.

A user may carry and/or be connected to one or more of such mentioned communication devices and/or other types of communication devices, in particular as disclosed herein.

## PTT control unit

The PTT control unit 105 may be an intelligent control box adapter with Push-To-talk (PTT) functionality such that communication to and from a connected headset 103 and/or one or more communication devices 109, 111 may be controlled via the PTT interface on the PTT control unit 105. The PPT control unit 105 may e.g. be in the form of a relatively small body-worn box e.g. to be attached to a vest, suit, or other of the user 101. The unit 105 may contain several interfaces, for connecting a headset 103 and one or more communication devices 109, 111, typically referred to as communication ports or simply "COM port". The PTT control unit 105 may contain a simple (e.g. stealth mode) user interface for controlling the operation of the headset 3 and/or connected communication devices 109, 111 in an easy and intuitive manner. Stealth mode means without any or at least little emission of visual or auditory feedback to the surrounding environment. The user interface of the PTT control unit 105 may e.g. be in the form of tactile buttons for controlling one or more devices (103, 105, 107, 109, 111) as worn/carried and used by the user 101. The user interface of the PTT control unit 105 may also control or communicate with other external devices via an intercom system/communication hub. The user interface of the PTT control box may for example contain two main PTT buttons on the side and two additional PTT buttons on the front for controlling respectively connected communication devices. The PTT control unit 105 may contain one or more buttons for controlling the connected headset 103 such that the different operation modes of the headset 103 may be activated, deactivated, or otherwise influenced.

The PTT buttons are typically configured to control the transmission of data and/or voice signals from the user 101 via one or more connected devices 109, 111 such that when one or more buttons are activated, the PTT control unit 105 is signalling one or more radios 109, 111 to start transmission.

An in-ear tactical hearing protection and communication system configured for dismounted operation according to an exemplary embodiment is illustrated in Fig. 1. A user 1, such as a soldier or public safety agent, is wearing a rugged in-ear hearing protection headset 103, such as the INVISIO X7, connected to a PTT control unit 105, such as the INVISIO V60 II ADP, comprising four dedicated PTT buttons, a mode button, three COMM interfaces and one headset interface. The user 101 may additionally be carrying (as illustrated in Fig. 1) an End-User-Device (EUD) 107 e.g. in the form of a chest mounted rugged casing holding a smart phone or tablet like the Samsung® S23 Galaxy Tactical Edition smartphone connected with the PTT control unit 105. The EUD 107 may e.g. be running a "battle management system" such as Android Team Awareness Kit (ATAK) for precision targeting, surrounding land formation intelligence, situational awareness, navigation, and data sharing via a smart phone touch screen interface. The user 101 may e.g. be a team leader in a squat or similar carrying a first radio 109, such as a portable Thales SquatNet® soldier radio, for communication between team members in the squad and a second radio 111, such as a Harris RF-7800M-HH, for voice and data communication with a connected (communication device or system of) a headquarter or similar. In the illustrated example, both the first 109 and second radio 111 is connected to the PTT control unit 105.

Fig. 2A schematically illustrates an example of a PTT control unit 105. The PTT control unit 105 may as an example comprise a rugged casing or housing 201 having four connection interfaces 203a-d, one 203a for connecting with a headset 103 and three 203b-d for respectively connecting to one or more of a plurality of communication devices e.g. such as 107, 109 and 111 of Figure 1 and elsewhere. The PTT control unit 105 may contain a main control unit (MCU) 205 such as a microcontroller system (e.g. ST from STMicroelectronics) on a chip (SoC) e.g. using an ARM processor core based on a Reduced Instruction Set Computing Architecture (RISC) or an FPGA processor or similar for executing instructions, controlling other units or elements of the PTT control unit 105, and/or performing complex computational tasks. The MCU 205 may be the central unit responsible for overall PTT control unit 105 control and coordination such as managing connection interface type determination, group configurations, and communication with the other processors, elements, and electronic circuits. The MCU 205 may e.g. execute desired/available functions of the software/firmware of the PTT unit 105 in accordance with connected devices to enable the functionality/behaviour of connected devices and the PTT unit 105, e.g. or preferably in response to respective cable chip settings of one or more connected devices. The MCU 205 may be in connection with a dedicated digital signal processing DSP unit 207 for advanced analog and/or digital signal processing.

The DSP unit may be a dedicated processor for handling audio mixing and processing tasks to ensure correct audio distribution across devices connected to the PTT control unit 105. The MCU 205 and DSP 207 processors may separately or in conjunction be configured to operate at least one artificial neural network engine configured to execute functionality such as one or more of speech recognition, voice-to-text, image classification, enhanced situational awareness, 3D directional sound processing, advanced signal processing, active noise cancellation, etc.

The processors 205,207 may be in connection with a memory component 211 for storing code, setting, instructions, and other relevant data. The MCU 205 may further be in connection with a USB hub element 213 or similar for administrating and negotiation of digital data protocols with connected devices with digital capabilities and with a power manager 215 controlling power routing via the PTT control unit 105, such as powering the PTT unit 105 itself and a connected headset 103 via an internal battery 217 and/or a connected radio 109,111 with power sharing capabilities, and/or directing power to or from a connected power bank or external power source to e.g. charge a radio 109 or 111 or the EUD 107.

The PTT control unit 105 may further contain a wireless module 219 such as a Blue-tooth transmitter or near-field wireless communication component for short range wireless communication with additional devices used by the user 101. The PTT control unit 105 may additionally contain a push-to-talk actuator module 221 handling activation events associated with push-to-talk (PTT) buttons 223a and 223b on the PTT control unit 105 in relation to the connected communication devices (107, 109, 111) so that a communication device 107, 109, 111 or headset 103 respectively can be operated via one or more push buttons on the PTT control unit 105. When a user activates the one or more of the physical PTT buttons 223a and 223b on the PTT control unit 105, a Carrier Operated Relay (COR) or Carrier Operated Switch (COS) signal or similar may be generated or triggered by the actuator module 221 and transmitted to the corresponding communication device 109 or 111 associated with the activated PTT button and additionally the microphone in the headset 103 may be unmuted. The COR/COS operates by generating a digital signal that switches between logic levels (typically +5V and Ground), indicating the activation of the transmitter by activating a squelch circuit or similar in the communication device thereby allowing the communication device to transmit a signal.

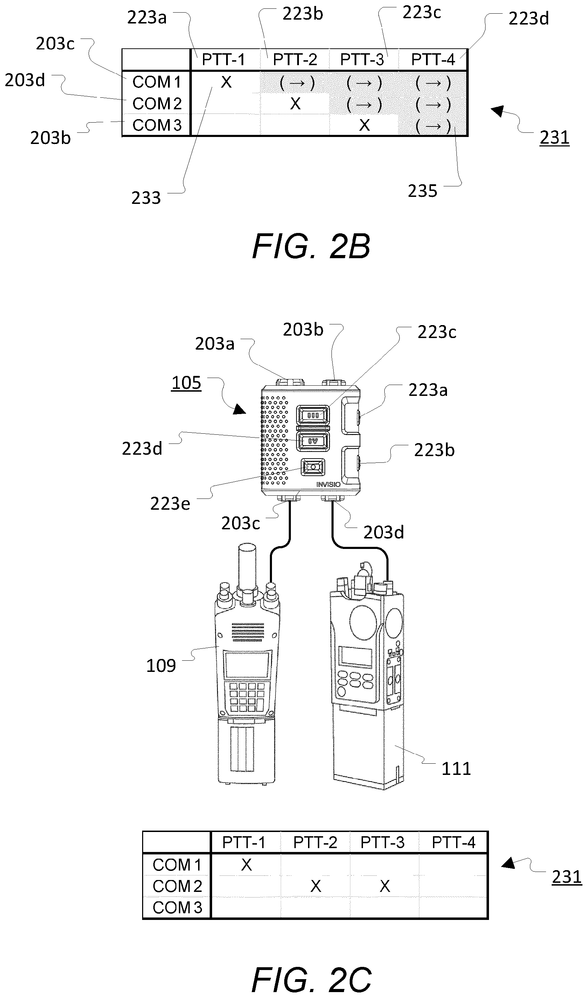

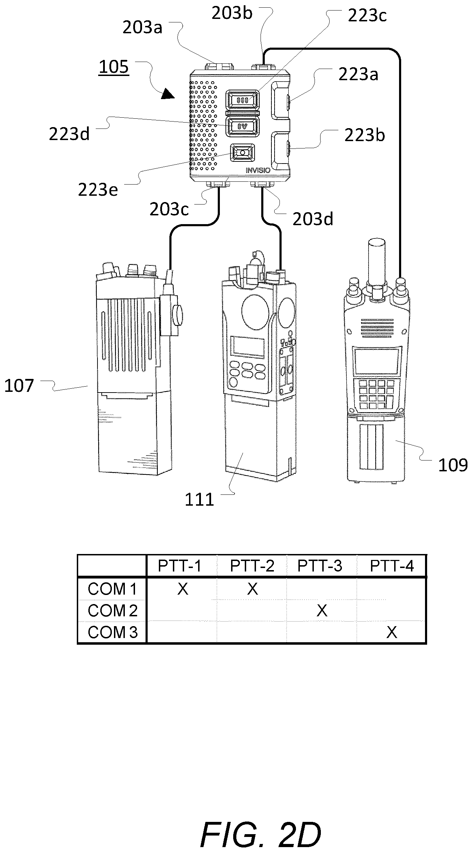

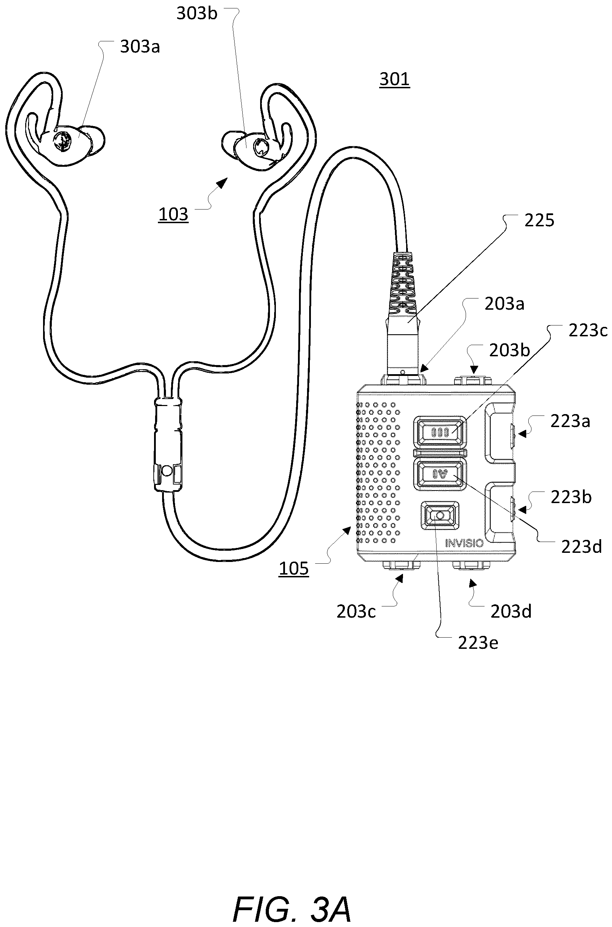

It is noted that the PTT control unit 105 may comprise further push buttons than illustrated in Fig. 2A, e.g. two additional PTT buttons on the front side, PTT-3 button (see e.g. 223c in Figs. 2C, 2D, 3A, etc.) and PTT-4 button (see e.g. 223d in Figs. 2C, 2D, 3A, etc.) as described elsewhere along with a dedicated "mode" button (see e.g. 223e in Figs. 2C, 2D, 3A, etc.).

When a communication device 107, 109, 111 and/or headset 103 is connected via a respective dedicated (e.g. male) connector 225 configured to interface with a respective (e.g. female) connection interface 203a-d on the PTT control unit 105, information and functional settings may be transmitted via the connection interface e.g. or preferably as described at least in part in EP2845115B1 (hereby incorporated by reference in its entirety) from a "cable chip" 227 embedded or located in the connector 225 (or alternative embedded or located in the cable of the connector 225 or elsewhere). The cable chip/microchip 227 may comprises an embedded memory storing data representing code, settings, instructions, and/or other data. When the connector 225 is connected to a respective connection interface 203a-d, the data (such as information, (configuration) settings, code, instructions, etc.) may be transferred directly from the cable chip 227 to the MCU 205 for configuration or other related tasks and uses of the connected device (as connected by the specific connector 225). Audio signal handling in the PTT control unit 105 may be facilitated by one or more CODEC modules 229.

The connector pair 203a-d and 225 should preferably be suitable for military and security applications, such as an ODU AMC® connector with between 3-55 contacts/terminals and be water resistant.

Inside the cable chip 227 (and/or an associated memory of the chip), a set of settings may be stored. When the memory is read, the settings may be transferred into the MCU 205 and distributed to the appropriate sections of the code and/or peripheral internal units such as the DSP 207, etc. The stored information/data of the cable chip may e.g. be organized as a list of "feature calls", also denoted "feature requests", followed by specific settings and e.g. other data respectively associated with the requested feature (i.e. the feature request). Thus, the cable chip 227 may store feature-requests (of/for the connected device) that when obtained by the MCU 205 will cause relevant processor(s) to execute instructions stored in the memory 211 of the control unit 105 corresponding to and/or carrying out the specific functionality associated with a requested "feature" using the specific set of settings and/or data transferred from the cable chip 227 for the particular feature. Examples of features may be:

- Audio Interfacing features, which may define an audio interface, impedance, gain, etc,
- Push-To-Talk / Protocol Interfacing, which may define protocols such as UART, USB, pulsing interface (e.g. by using a variable resistance between terminals and Ground to signal different actions by generating voltage pulses. For example, shorting a particular microphone terminal to Ground for signalling to a connected phone e.g. to answer/hangup/take picture, etc.), etc.,
- Control functions (e.g. enabling specific functionality based on conditions such as VOX ("voice-operated exchange"), etc.),
- UI definitions (which may define short/long press of PTT button functionality or mode button functions and key combos (combination of different simultaneous button presses),
- Various different audio algorithms, and
- Audio routing (that may adjust the flow of the audio including multi headset setup, crossbanding (relay) between connected radios, etc.) and side tone in headset.

The cable chip 227 may additionally or alternatively be used for device authentication and/or for specific user rights or accesses associated with the specific cable and/or the connected device connected by the specific cable.

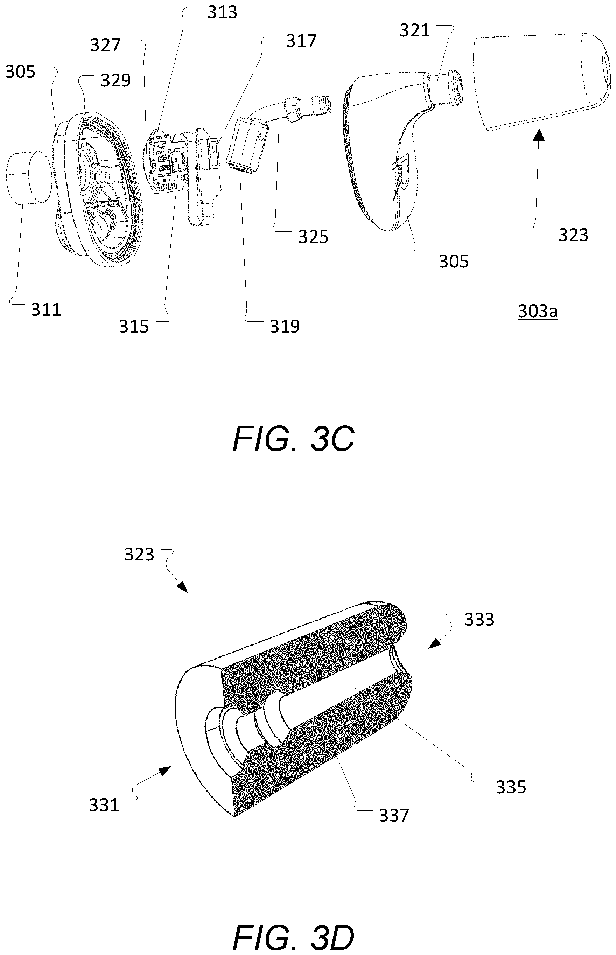

Thus, the PTT control unit 105 may be configured to operate both data and voice communication channels on connected communication devices 107, 109 and 111 in response to the user speaking (VOX) and/or pushing one or more buttons 223a, 223b on the PTT control unit 105. Based on the particular configuration of devices illustrated in Fig. 1 (i.e. setup e.g. like their number, type, and/or respective connections), the personal in-ear tactical hearing protection and communication system (e.g. see Fig. 1) may be configured such that communication is handled in the following manner. Communication from the first radio 109 (e.g. receive or "Rx") is directed to the user 101 via loudspeakers in both the left and right ear of the headset 103. Communication from the user 101 (e.g. transmit or "Tx") via the first radio 109 may be activated by the voice of the user referred to as "voice-operated exchange" or "VOX". In a voice activated transmission configuration (VOX), the PTT control unit 105 may be configured to process signals obtained by a transmit (Tx) microphone (see e.g. 317 in Fig. 3C) dedicated for user voice communication in the headset 103, such that when the user 101 is speaking, the MCU 205 may process the sound signal from at least the microphone 317 to detect a speech signal and in such cases thereby recognising an activation event and the MCU 205 may thus signal the PTT actuator module 221 accordingly. In response to the activation event, the PTT actuator module 221 may send an electrical signal to a PTT circuit in the first radio 109 triggering the radio to activate a transmission mode thereby allowing the user to transmit a voice signal via the radio 109. The second radio 111 may e.g. be configured in a dual net operation, such that voice communication can be performed via two separate channels or frequency bands simultaneously. The PTT control unit 105 may be adapted such that two PTT-buttons 223a and 223b on the PTT control unit 105 may respectively be assigned to transmitting a voice message via the respective two separate nets, a first net "net 1" and a second net "net 2". In response to the user activating the first PTT-1 button 223a by pressing the button physically, the PTT actuator module 221 may register an activation event similar to the VOX situation but associated with a PTT-1 button 223a and thereby signalling a PTT circuit in the second radio 111 associated with net 1, as described elsewhere. Similarly, the second PTT-2 button 223b may be used to transmit a voice signal via net 2 on the second radio 111.

Accordingly, the PTT control unit 105 may enable the user to communicate via a specific communication device by performing an action, such as pushing a button or starting to speak (VOX), where this action may also be referred to as latching in on a communication channel. Voice communication via the EUD 107 may e.g. be controlled via two additional push-buttons (not shown, see 2C and 2D), one button associated to digital "picking up call" signalling from the PTT control unit 105 to the EUD 107 in relation to receiving a cell phone call and one button for "hang up call" for ending a call. Different push-button combinations ("key combos") may be used for more advanced actions, such as transmission of specific data or configuring the way the loudspeakers in the headset should emit sound, such as left/right ear only, mute all communication, mute single communication channel, etc.

The PTT control unit 105 may additionally or alternatively contain instructions related to assigning PTT buttons 223a and 223b and functionality following a hierarchical scheme or other suitable scheme. Depending on the type of connected communication device, one or more buttons may be requested (e.g. as part of the information stored in the cable chip 227) to support the communication capabilities of the connected device such as two buttons would be requested if a dual net ratio is connected and three buttons in case of a tri net interface, etc. This is advantageous in combination with the cable chip functionality, as a default scheme may be applied if the cable chip data fails to specify unique instructions thereby providing a system with an intuitive default configuration behaviour across communication platforms.

A special set of PTT allocation rules may be applied by the PTT control unit 105 to facilitate easy and intuitive operation and a dynamic use. Individually connected communication devices 107, 109 and 111 may request a number of PTT buttons to operate the respective device via the simple push button interface on the control unit 105. As a limited number of physical buttons are available on the device 105, a negotiation scheme may be implemented for PTT-button allocation.

An example of a PTT-button allocation scheme 231 is schematically illustrated in Fig. 2B. The PTT-button allocation may follow an intuitive hierarchical structure, where buttons 223a-d are assigned to respective communication devices 107,109,111 following a prioritized scheme depending on which connection interface 203b-d a communication device is connected to. One of the "X"s 233 in the allocation scheme 231 show that a communication device 107,109,111 connected to connection interface 203c designated "COM 1" (in Fig. 2C pointing downwards and to the right as seen from the users 101 point of view while it is left on the illustration), when the PTT control unit 105 illustrated in Fig. 2A, etc. is worn by a user 101) will be assigned to use PTT-1 button 223a for a primary communication channel of the communication device connected to connection interface 203c. Likewise, a communication device 107,109,111 connected to connection interface 203d designated "COM 2" (in Fig. 2C pointing downwards and to the left as seen from the users point of view while is right on the illustration) when the PTT control unit 105 illustrated in Fig. 2A, etc. is worn by a user 101) will per default be assigned to use PTT-2 button 223b (as indicated by the appropriate "X" in Fig. 2B) for operating the primary communication channel for the communication device connected to connection interface 203d and lastly a communication device 107,109,111 connected to connection interface 203a designated "COM 3" (i.e. pointing upwards next to the headset connector 203a when the PTT control unit 105 illustrated in Fig. 2A, etc. is worn by a user 101) will per default be assigned to use PTT-3 button 223b (as indicated by the appropriate "X" in Fig. 2B) for operating the primary communication channel for the communication device connected to connection interface 203a. The "(→)"235 in Fig. 2B indicate how the allocated PTT button 223a-d may be re-assigned when/if additional communication channels are requested by connected communication devices 107,109,111, where an example is given in the following.

In addition to the allocation scheme 231, one or more rules may be applied to ensure easy, intuitive and clear communication via one or more connected communication devices 107,109,111.

The rules may as an example e.g. be:

Rule 1: All connected devices, being communication devices 107, 109 and 111 and/or non-user specific radios, are allowed a minimum of one PTT button if requested by the device/cable.

Rule 2: PTT allocation is prioritized according to port number 203b-d (when port number 203a is used/to be used by a headset according to the allocation scheme 231), i.e. 203b is prioritised before 203c, 203c before 203d, etc.

Rule 3: When a tri-net Interface (for a communication device having three dedicated communication channels) has highest priority and another multi-net communication device is connected, one of the nets of the tri-net will be given up (i.e. no dedicated PTT button is no longer assigned to the net given up).

Rule 4: When a quad-net interface has highest priority and another multi-net interface is connected, two of the nets of the quad-net will be given up (no dedicated PTT button is assigned any longer to the two nets given up) to allow more than one net on another multi-net interface to function.

In case additional wireless remote PTT buttons are connected, additional rules may be applied such as: A five-button wireless remote PTT (see e.g. 113 in Fig. 1) may mimic the entire control unit 105 thereby expanding the number of available PTT buttons or mirroring the buttons on the PTT control unit 105.

To illustrate the PTT allocation functionality by way of an example, Figs. 2C and 2D schematically illustrate two different configurations of communication devices connected to the PTT control unit 105 and the corresponding PTT button allocation scheme 231.

Fig. 2C illustrate a configuration where a first communication device 109 (being as an example a L3Harris AN/PRC-163 Multi-channel Handheld Radio) is configured for single channel communication configuration and is connected to the "COM 1" interface 203c of a PTT control unit 105 and a second communication device 111 (being as an example a Thales AN/PRC-148D IMBITR 2-channel radio) configured for multi-channel, or more specifically for dual channel, communication configuration and is connected to the "COM 2" interface 203d of the PTT control unit 105. The corresponding PTT button allocation scheme 231 is additionally displayed in Fig. 2C and illustrate that the single communication channel of communication device 109 is activated using the PTT-1 button 223a, the first communication channel of communication device 111 is activated using the PTT-2 223b button, and the second communication channel of communication device 111 is activated using the PTT-3 223c button. In the configuration displayed in Fig. 2C, all available communication channels are assigned to a respective PTT-button on the PTT control unit 105 as the number of requested communication channels does not exceed the number of available buttons.

Fig. 2D illustrate a modified (compared to Fig. 2C) configuration where the first communication device 109 (still being a L3Harris AN/PRC-163 Multi-channel Handheld Radio) is configured for single channel communication configuration but is now being connected to the "COM 2" interface 203d of a PTT control unit 105 and where the second communication device 111 (still being a Thales AN/PRC-148D IMBITR 2-channel radio) is now connected to the "COM 3" interface 203b of the PTT control unit 105. A third communication device 107 (being as an example a Persistent Systems MPU5 network radio, operating the Wave Relay® MANET solution) is configured with three individual channels for voice and/or data transmission that is connected to the "COM 1" interface 203c. The corresponding modified PTT button allocation scheme 231 is additionally illustrated in Fig. 2D and show that in this configuration, (in fig. 2D) the number of requested communication channels across the connected communication devices 107,109,111 exceeds the number of available PTT buttons on the PTT control unit (as an example, six available nets vs. four available PTT buttons). This requires that the PTT control unit 105 may utilize the one or more additional rules. Rule 3 will be applied, such that one of the communication channels of both the third 107 and the second 111 communication device will not be assigned to a PTT button to obey Rule 1 and thereby allowing the user (see e.g. 101 in Fig. 1) to operate at least the primary communication channel on each of all the connected communication devices directly via a dedicated PTT button which is advantageous in demanding environments as clear communication via a plurality of different communication devices may be required.

In one embodiment, the one or more processors, electronic circuits, and logical components configured to execute desired/available functions of the embedded software/firmware (as explained by some examples in the previous) comprised by the PTT control 105 may constitute a communication module 237 (see e.g. Fig. 2A). Thus, the communication module 237 may be configured to receive an audio signal from a communication device 107, 109, 111 (e.g. or preferably a radio 109, 111) and provide the received audio signal, or a processed version thereof, to the user 101, more specifically to the inner ear of the user 101.

In-ear Headset

In-ear tactical hearing protection and communication headsets are generally providing a higher level of hearing protection then a circumaural headset variant. Additionally, full situational awareness of the surroundings may be achieved by using in-ear devices instead of over-the ear types, as an in-ear device does not obstruct the natural geometry of the ear, thereby allowing the user to determine the direction of incoming sound almost as precisely as with the naked ear. This provides a clear advantage when used in demanding environments, such as a riot control operation by a police officer or on a battlefield by a solider to precisely pinpoint the direction of voice or sound. To achieve crystal clear communication even in demanding and high noise environments in-ear headsets may utilizes bone conduction for obtaining and transmitting a voice signal from the user to team members via a land mobile radio (LMR) for example. This means that instead of picking up air-borne vibrations like traditional microphones, bone conduction microphones pick up vibrations directly from the user's jawbone when speaking. This method allows the in-ear headset comprising a bone conduction microphone to deliver clear communication under extreme noise conditions as ambient external environmental noise are automatically excluded. Examples of such a solution is known from the INVISIO X5 in-ear headset using a more traditional microphone transducer modified with a rubber dome or bladder to transfer a jaw vibrations signal from the inner part of the ear into an electrical voice signal as at least described partly in document EP3298800B1. However, the nature of bone conducted speech can lead to sound degradation due to the lack of high frequency components in the speech signal, resulting in a muffled effect. That degradation, combined with typical aggressive audio compression in tactical radios, can lead to compromised quality when it comes to narrowband wireless RF voice communication via radios and other. Additionally, the fitting of the in-ear hearing protection and communication headset may be very significant for the performance of the in-ear headset. If the bone conduction microphone is positioned non-optimally with less, little, or even no contact to the tissue of the ear canal, speech from the user will typically severely be deteriorated or not obtained at all. Furthermore, the otherwise superior hearing protection ability of an in-ear device rely on the fitting into the ear canal to a high degree making it very important that the in-ear device is mounted precisely in the ear-canal and with a proper fit for providing the seal to the ambient environment.

Figure 3A schematically illustrate an example of an improved in-ear tactical communication and hearing protection system 301 comprising an in-ear tactical communication and hearing protecting headset 103 connected to a PTT control unit 105 (e.g. or preferably as disclosed herein) via a cable and a connector 225. The in-ear tactical communication and hearing protecting headset 103 comprising a pair of earpieces 303a, 303b adapted to be mounted inside the right and left ear canal, respectively, of the user when worn to provide hearing protecting, situational awareness, and clear and undistorted communication via one or more communication devices (see e.g. Fig. 1 and elsewhere).

The headset 103 may be configured to receive and transmit audio and data signal via the wired cable and connector 225. The headset comprises two similar earpieces, left 303b L and right 303a R. Both earpieces contain similar components and may provide similar functionality. Even though the following description may refer only to the schematics, components, and functions of one earpiece, it should be understood that similar or identical components are present in the other, and furthermore, functionality may be achieved when both earpieces 303a,303b work in conjunction.

Each of the earpieces may contain a plurality of transducers for obtaining and emitting acoustic signals to the user and potentially other sensors such as an optical heart rate monitor. Each of the transducers are communicatively in contact with the PTT control unit 105 via the cable and connector 225 for audio and data exchange, signal processing, and supply of power. When the headset 103 receives an audio signal (Rx) via a connected communication device 107, 109,111, (e.g. see fig. 1 and elsewhere), the audio signal may be directed and emitted to the ear canal of the wearer via a loudspeaker or similar (see e.g. 319 in Fig. 3C) inside each of the earpieces 303a,303b. When the wearer is talking, the speech signal may be obtained and transmitted (Tx) via a radio, through the PTT control unit 105, by one or more dedicated respective transducers 315, 313 (see e.g. 315 and 317, respectively, in Fig. 3C).

The headset 103 may have a set of ambient microphones or similar (see e.g. 315 in Fig. 3C), one on each earpiece 303a, 303b pointing outwards from the wearers head when mounted, configured for picking up ambient sounds, which may then be processed in real-time by the PTT control unit 105 (and/or by the in-ear headset 103 itself in case the headset comprises one or more processors as described in relation to Fig. 2A, describing processors in the PTT control unit 105) before the sound signals are emitted to the wearer via the loudspeakers. This provides the effect of making the headset "transparent" in terms of listening to the surroundings thereby simultaneously providing situational awareness and hearing protection. Situational awareness should be understood as the ability to hear and/or perceive the surroundings and determine the direction of sounds. Thus, the in-ear tactical communication and hearing protection system 301 may be able to operate in this active mode of operation when the system 301 is powered.

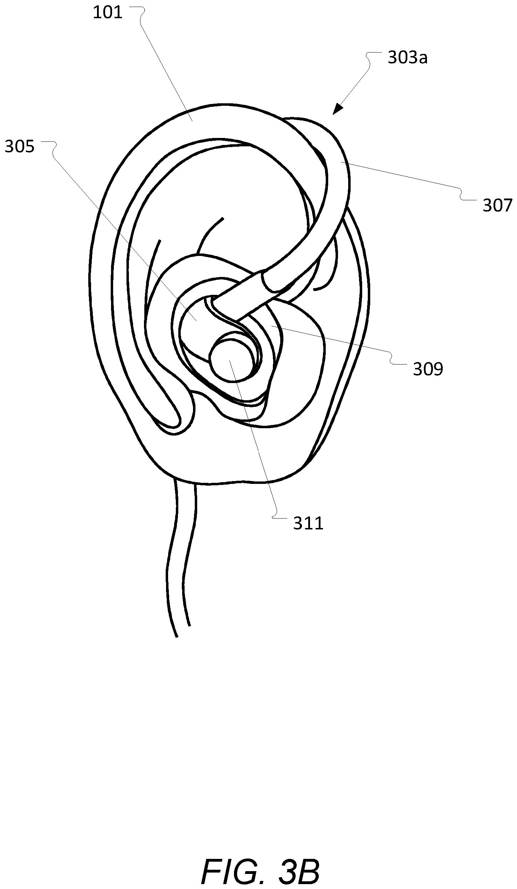

Fig. 3B schematically illustrates an example of an embodiment of the in-ear tactical communication and hearing protection headset 103 as disclosed herein when fitted properly inside the ear-canal of a user 101. Fig. 3B show the Right 303a R earpiece fitted into the right ear of the user 101. When securely fitted in the ear-canal of the user the in-ear piece expose parts of the enclosure or shell 305, an ear-hook 307 or similar for providing cable guiding and supporting a secure fit in the ear and parts of a silicone sleeve or other sealing element 309 is visible and used as a supporting layer between the shell 305 and the ear tissue of the user 101 for improved comport when worn. Additionally, a wind filter 311 is shown in more or less the centre of the earpiece covering an ambient microphone (see e.g. 315 in Fig. 3C) for mechanically removing or reducing turbulent and/or static wind noise. As seen from Figu. 3B, the entire personal ear geometry is exposed to the ambient environment providing a natural structure for pinpointing or registering the direction and origin of an incoming sound signal compared to conventional "over-the-ear" headsets thereby providing an improved directional determination of a sound source via the ambient microphone (see e.g. 315 in Fig. 3C) when a situational awareness mode is activated (e.g. by powering the system 301 on) since the in-ear headset 103 do not obstruct the natural structure of the ear and register sound at a location at or near the entry of the ear canal of the user 101.

Fig. 3C schematically illustrates an example of an exploded view of the right 303a R earpiece of the in-ear tactical communication and hearing protection headset 103 of Fig. 3A. A main (flexible) printed circuit board 313 (PCB) is supporting electronic components and circuits of the earpiece 303a while being enclosed by a shell or housing 305. When the earpiece 303a is assembled, it is configured to be submersible in water up to at least 1 meter, and be dust and sand-proof, e.g. according to a suitable specific ingress protection (IP) rating, e.g. an IP68 rating, to withstand hash conditions of demanding environments.

In some embodiments, the PCB 313 may contain the communication module 237 (e.g. one or more processors and/or other logical components) as discussed in relation to the PTT control 105 unit and elsewhere and may then carry out functionality as described in relation to these (either instead or in addition or as a supplement).

The main functionalities of the headset, i.e., listening to surroundings (situational awareness) and transmitting and receiving sounds, are enabled by three main transducer components: The ambient microphone 315, the transmit (Tx) microphone 317, and the loudspeaker 319.

The speaker unit 319 (i.e. loudspeaker) is used to convert an electric audio signal into an acoustic signal to play audio into the user's ear. The sound signal may comprise both the ambient sounds from the user's surroundings received by the ambient microphone 315 and received radio signals (Rx) in case the PTT control unit 105 is connected to one or more communication devices.

In order to minimize of the overall size of the earpiece 303a, it may be an advantage to utilize a balanced armature driver as a speaker unit 319, as they have a very small formfactor and ability to produce a fairly high sound pressure level relative to their size.

The speaker unit 319 in earpiece unit 303a may be directly and/or dedicatedly wired to the PTT control unit 105 such as either via a direct dedicated connection line through the cable and connector; see e.g. 225 in Fig. 3A, or alternatively indirectly via the flex PCB 313 sharing connection lines with the PCB 313 to the PTT control unit 105 or internal processors and other logical components.

The speaker unit 319 may be mounted in the earpiece 303a in such a way that the sound is guided via a "spout" or "funnel" 321 structure of the earpiece 303a shell, pointing into the ear-canal of the user 101 when the earpiece 303a is worn. The shape and size of the spout or funnel 321 may affect the sound received by the user, and the spout or funnel 321 may also serve as a coupling mechanism for an ear tip 323 that may be mounted as an extension of the shell 305 and forming part of the earpiece 303a.

The transmit (Tx) microphone(s) 317 employed in the headset 103 may be an accelerometer type vibration sensitive transducer. When the speaker unit 319 produce a sound signal (e.g. when outputting an incoming radio message), it may cause the speaker unit 319 to vibrate slightly, which may induce an effect called "crosstalk" between the speaker unit 319 and the transmit (Tx) microphone 317 as the Tx microphone 317 may register the vibration of the speaker unit 319. Crosstalk refers to unintended transmission of audio signals between communication channels or circuits, which is undesirable in tactical communication where sensitive or confidential messages may be transmitted. To decrease the vibrations produced by the speaker unit 319, the chosen transducer type is advantageously a so called "dual" balanced armature driver, in which two identical drivers are mounted together, up against each other, to cancel out the vibrations that each driver produces as they will be operating in antiphase.

Another way to decrease the vibrations transferred between the speaker 319 and the rest of the headset earpiece 303a may be by vibrational decoupling between the speaker unit 319 and the earpiece shell 305 and other internal components. This may e.g. be achieved by two methods. First, the electrical wires between the speaker unit 319 and the rest of the earpiece 303a may be so called "litz" wires, which are very thin and flexible, ensuring a low amount of vibration transfer. Secondly, the speaker unit 319 may be inserted and mounted into a rubber-sleeve or similar 325. The rubber-sleeve may be designed to both hold the speaker unit 319 and to provide a sound bore for directing the acoustic signal to the sprout or funnel 321. The rubber-sleeve may thereby function as a suspension with only a single point of contact with the rest of the headset (i.e., at the sprout or funnel 321 of the earpiece shell 305). The rubber-sleeve may thereby advantageously make the speakers "float" or freely hang inside of the earpiece 303a, such that the speaker unit 319 is not in direct physical contact with the flex PCB 313 and associated components 315, 317 at any point.

The ambient microphone unit 315 may comprise a so called "MEMS" microphone, which has a small formfactor. Since the ambient microphone 315 may be a small flat component, it may be possible to mount the microphone directly on the flex PCB 313. The ambient microphone is positioned such that the microphone port or active side is pointing outwards from the headset, almost perpendicular to the head of the user when worn (see e.g. Fig. 3B). Inside the earpiece 303a, a flexible membrane 327, e.g. a rubber membrane, is placed in front of the microphone port (i.e. in front of the active side) to protect components inside the earpiece 303a from dust and debris and to achieve submergibility in water. On top of the membrane is a grid, grille, or similar 329 (e.g. forming part of the shell 305) to further protect the microphone-membrane assembly from damage. On the grid, etc. 329, a circular porous foam wind filter 311 is placed to reduce wind noise otherwise affecting the microphone, which is advantageous when operating the headset 103 in high wind speeds such as sailing in a RHIB (rigid-hulled inflatable boat) across the water or in other windy environments.

The main purpose of the ambient microphone 315 is to act as the artificial hearing of the user when wearing the headset 103. The ambient microphone 315 receives sound from the surroundings around the user and provides an audio signal in response thereto, which can then be transmitted to the user via the speaker unit 319 after being processed in the PTT control unit 105 (and/or the headset) so the user can hear the surroundings thereby providing situational awareness. A secondary purpose of the ambient microphone 315 may be to measure the sound pressure level of the external ambient sounds so that the "active hearing protection" algorithm (e.g. executed by the MCU 205 and DSP 207 processors of the PTT control unit 105; see elsewhere) can process and adjust accordingly. It should be understood that "active hearing protection" (AHP) is a form of active noise control, carried out via the headset transducers 315, 319 and the electrical circuitry of the PTT control unit 105, in which ambient sound is allowed to be transmitted to the user while limiting its overall amplitude so as to protect the user's hearing. This feature provides the user with situational awareness by enabling the user to hear ambient sounds while protecting the user's hearing from overly loud and potentially damaging sounds such as heavy machinery, a gunshot, etc.

In the in-ear tactical communication and hearing protection headset 103, the ambient microphone 315 may additionally be used to support the Tx microphone 317 when creating a clear communication signal to be transmitted via a communication device (e.g. see Fig. 1 and elsewhere) in situations with a varying ambient noise level. This is advantageous as a speech signal obtained by the ambient microphone 315 may have a significantly higher quality than the Tx microphone 317 in low ambient noise. By dynamically combining the input signal from the Tx microphone 317 and the ambient microphone (i.e. preferably from both earpieces R 303a and L 303b), the voice of the user may become gradually clearer and more undistorted, when the external ambient noise level is low, since the ambient microphone 315 may "take over" or provide the dominant contribution in low ambient noise situations and vice-versa in high ambient noise situations. The voice signal mixing between the ambient microphone(s) 315 and the Tx microphone(s) may be handled by the PTT control unit 105.

The main purpose of the Transmission (Tx) microphone 317 is to obtain the voice signal of the user when speaking so the voice signal can be forwarded to the PTT control unit 105 and subsequently be transmitted to a remote receiver via other equipment such as connected communication devices 107, 109, 111 or an intercom system, e.g. or preferably an intercom system as disclosed in European patent applications number 24174205.5, 24182433.3, and 24189349.4 (all hereby incorporated by reference in their respective entirety) respectively disclosing embodiments of an intercom system and aspects thereof.

An important functionality of the Tx microphone 317 may be to ensure clear and undistorted communication by obtaining only the voice of the user (at least to a large or an optimal extent) to be received and not obtaining external ambient noise (at least to a large or an optimal extent) from around the user even in high noise environments. One way to achieve this is by using so called bone-conduction microphones (BCM) e.g. as know from the INVISIO® X5 headset. However, some types of BMC microphones may be relatively difficult to fit properly in an ear canal of the user, which may cause deterioration of the voice signal. Therefor a different approach is applied in the tactical communication and hearing protection headset 103 as disclosed herein by utilizing (at least in some/preferred embodiments) a vibration pick-up sensor (VPU) as the Tx microphone 317.

The Tx microphone 317 may accordingly function as an accelerometer picking up the vibrations from the pinna (e.g. outer ear structure) of the user when speaking. The Tx microphone 317 may be a component comprising a MEMS microphone with a tiny mass attached to an internal membrane thereby converting the microphone into an accelerometer, particularly when the microphone port hole/surface is closed (e.g. no air borne acoustic signal can reach the active surface of the MEMS microphone).

In existing types of BCM microphones such as the ones known from the INVISIO® X5 headset, the Tx microphone is a standard type microphone (e.g. air borne acoustic signal to electric signal transducer) with a small rubber dome on top of the sensing area, which thereby may be modified to pick up vibrations and converting them into varying sound pressure fluctuations in front of the microphone port (e.g. as partly described in document EP3298800B1). This type and similar BCM microphones may be quite sensitivity to the actual placement in the ear of the user since the rubber dome must have firm contact with the user's ear to ensure good vibration transmission. It is therefore advantageous to utilize a VPU component as the Tx microphone 317, which does not rely on firm physical contact with the user's ear (canal). Rather, the (VPU) Tx microphone 317 may be placed as an internal component in the earpiece 303a as it is much less sensitive to the fit of the earpiece 303a in the ear. As long as the earpiece 303a is positioned firmly in the ear-canal of the user, a good vibration transfer will be achieved and hereby a good voice signal may be obtained by the VPU type Tx microphone 317.

The Tx microphone 317 may be placed inside the earpiece 303a as close as possible below the part of the shell 305 that is resting against the ear-canal of the user 101 when worn to maximize the vibration transfer from the user to the earpiece 303a when speaking. Furthermore, the Tx microphone 315 may be oriented such that the most sensitive axis of the sensor is perpendicular or substantially perpendicular to the ear canal of the user when worn. This may result in the highest signal output in response to obtaining speech with the flattest frequency response such that resonance contributions are minimized while also being less sensitive to external noise.

The (VPU) Tx microphone 317 may be a digital component whereas other components may be analog, such as for example the speaker unit 319. To be able to interface the Tx microphone 317 in an analog signal architecture, for example using analog audio signal exchange with the PTT control unit 105, it may be advantageous to convert the digital signal to an analog signal. This may be achieved in multiple ways involving a digital-to-analog converter component as generally known. However, a dedicated DAC circuitry 349 configured to perform a lossless front-end digital to analog signal conversion may advantageously be applied.

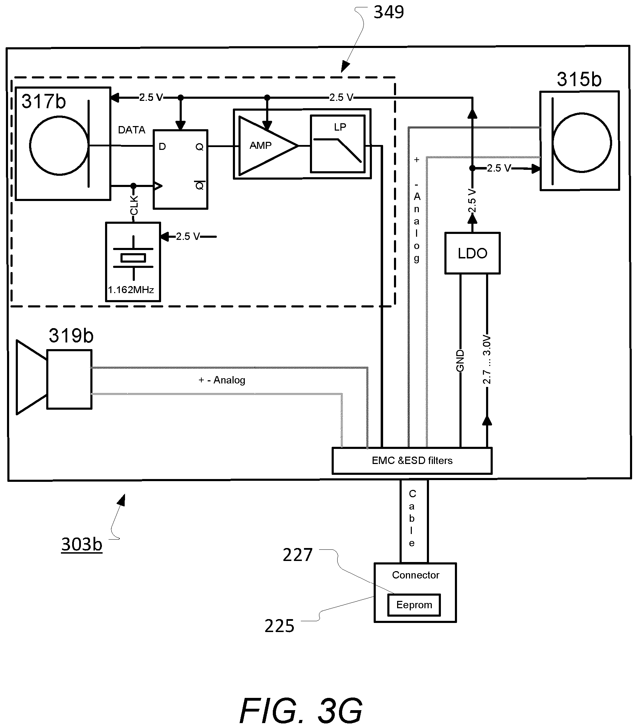

Fig. 3G schematically illustrates an example of a circuitry 349 located in the earpiece of the in-ear tactical communication and hearing protection headset of FIG. 3A configured to perform a lossless front-end digital to analog signal conversion of a digital output signal from Tx microphone 317.

As mentioned, the Tx microphone 317 may be a Digital VPU (Voice Pick Up) Microphone type, which output for example a digital Pulse Density Modulation (PDM) signal. For subsequent signal handling in an analog processing framework, each earpiece R 303a and L 303b may advantageously have a direct Digital to Analog signal conversion (DAC) circuit 349 on the PCB 313 configured to performi a lossless front-end conversion of the digital signal to an analog signal. An example of the DAC circuitry 349 in the Left earpiece 303b is shown in fig. 3G. The DAC circuitry 349 may comprise a D-FlipFlop and a 4'th Order Active Filter with a Bessel function for providing the best possible Analog Audio conversion compared to other mathematical functions such as e.g. Eliptic, Chebychev, Butterworth, etc. The converted Analog speech signal may be routed via a down-lead cable and connector 225 to the headset port 203a on a PTT control unit 105 for further Audio Processing before being forwarded to one or more connected communication devices.

To ensure an acceptable level of EMC protection, the earpiece 303a may utilize a metalized housing. The metallization is achieved by coating the inner part of the shells 305 in a thin electrically conductive layer, such as a thin metal layer. Both of the shell parts 305 may be metallized and connected to each other at the rim around the edge of the shells 305. The metalized housing may also be connected to a drain-wire that also acts as shielding in the headset 103 cable and connector 225.

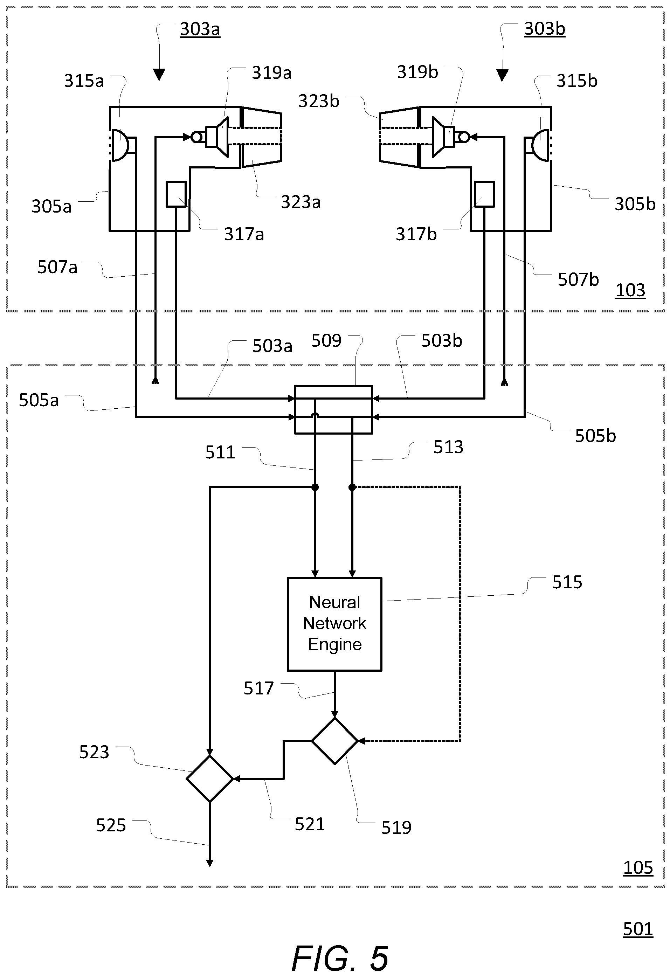

Due to the vibration-sensitive nature of the Tx microphone 317 certain challenges also arises, such as scratching noise from cord movement of cables on the body of the user 101 due to physical movement (e.g. turning, running, jumping, crewing etc.) and/or wind noise caused by the wind making the headset 103 and/or cables shake thereby causing the Tx microphone 317 to pick up noise. These challenges may at least be alleviated by signal processing algorithms ensuring that the signal-to-noise ratio is kept at least at an acceptable level even when these types of noises are introduced by for example performing a machine learning based voice signal filtration method with a speech enhancement element as explained in more details elsewhere (see e.g. Fig. 5 and elsewhere).

In further embodiments, the earpiece 303a may be provided with an additional microphone unit and/or loudspeaker unit positioned to monitor the in-ear space (e.g. between the eat-tip 323) and the inner part of the users ear) used in combination with a dedicated circuitry to provide active and/or automatic noise cancelation (ANC) and/or active noise reduction (ANR). The ANR/ANC functionality may use one or more dedicated electronic circuits (e.g. a part of the flex PCB 313 and/or PTT control unit 105) to generate anti-noise signals (e.g. via the speaker units 319a, 319b or the additional loudspeaker unit) that destructively interfere with ambient sound to cancel it thereby providing an improved hearing protections compared to utilizing only passive sound attenuation. Thus, ANC/ANR is a form of active noise control in which certain selected ambient sounds, that may be repetitive, are filtered out so that other sounds, such as radio communications, can be heard more clearly. As one example, a user riding in a helicopter may configure (e.g. via one or more button combination activations) the tactical communication and hearing protection system 301 to attenuate the repetitive sound of the helicopter engine and rotor while not attenuating desirable sounds such as speech and audible warning signals. By providing the earpiece 303a with an additional microphone for ANC/ANR, the overall hearing protection performance may be enhanced (and e.g. provide additionally 4-5 points to the SNR rating of the headset 103) compared to relying on passive hearing protection alone. Hearing protection may generally be quantified or represented by a SNR value being a Single Number Rating (SNR) system as per the International Organization for Standardization's ISO 4869 certification. The implementation of an additional loudspeaker may be embodied by fitting an additional loudspeaker transducer into the existing rubber sleeve 325 or preferably by adapting the interior of the earpiece 303a to fit a second additional separate rubber sleeve adjacent to the first rubber sleeve 325 so the acoustic performance of the loudspeakers may be preserved and engineered individually for providing optimal or enhanced performance. Additionally, or alternatively, an additional microphone may be placed in acoustic connection with the sprout or funnel 321 and/or rubber-sleeve for performing a seal-test to enable a check of whether the earpiece 303a is properly fitted prior to exposing the wearer to the demanding environment with potentially damaging noise levels.

The maximum level of passive hearing protection provided by the tactical communication and hearing protection headset 103 may be achieved when operating the headset 103 in a "passive noise control" mode, which occurs when the electrical circuitry of the headset 103 is turned off or if no ANC/ANR functionality is available, thereby relying only on the earpieces 303a R and 303b L to physically block sound-waves from reaching the eardrum of the user 101. Otherwise, a maximum level of hearing protection may be achieved having the ANC/ANR circuitry operating, as previously described, thereby relying on passive blocking in combination with anti-noise generation. As aforementioned, the level of hearing protection may be quantified by means of a SNR value according to the ISO 4869 standard measured in dB (decibel). The tactical communication and hearing protection headset 103 provided in this disclosure is configured to provide at least 30 dB SNR. A main factor for achieving a high level of hearing protection is the ear tip 323 configured to be mounted on the spout or funnel 321 of the shell 305. Ear tips for in-ear headsets are generally well known in the art and may be embodied in many different variations such as tri-flange silicone ear plugs like the SureFire™ EP4 Sonic Defenders Plus or foam type ear plugs like the Comply™ 400 Series Foam Ear Tips. However, it has been realized that some specific properties for a foam-type ear tip 323 are specifically advantageous for achieving improved performance for an in-ear tactical communication and hearing protection headset 103.

## Ear tip

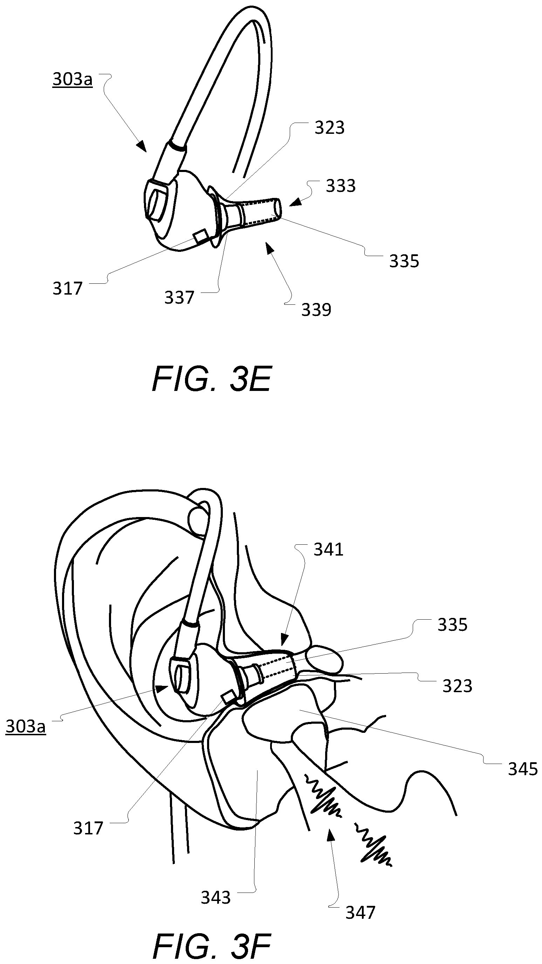

Fig. 3D schematically illustrates an example of a cross-sectional view of the ear tip 323 of the in-ear tactical communication and hearing protection headset 103 of Fig. 3A. In some embodiments, the ear tip 323 may have a distal end 331 facing the shell 305 of the in-ear device (e.g. 303a and/or 303b) and a proximal end 333 in the opposite direction facing the user 101 when wearing the headset 103. The ear tip may contain a sound bore 335 as an inner core of the ear tip 323. The sound bore 335 may be configured as an acoustic channel or tube for directing an air borne audio signal (e.g. generated at least by the loudspeaker 319) to the user 101. Thus, the sound bore may preferable be made in a semi-rigid material being substantially non-compressible such that the sound bore is unobstructed at all times providing free passage for acoustic signals to be transmitted. The sound bore may be made of plastic and have a structured region in the portion at its distal end 331 and be configured to engage with the spout 321 of the in-ear shell 305, such that the ear tip 323 may locked into place in relation to the shell of the headset 103. The body 337 of the ear-tip 323 may advantageously be made in user compressible material such as a foam-type material. Generally, foam-type ear tips work by first being compressed or squeezed together (e.g. into a compressed state, see e.g. 339 in Fig. 3E), whereafter the compressed foam earplug is positioned right away in the ear canal and allowed to relax so the foam will re-expand again and adapt firmly to the surrounding ear canal of the wearer (e.g. into a predefined less compressed state, see e.g. 341 in Fig. 3F) thereby creating an acoustic barrier for external sounds to reach eardrum of the user 101. Figure 3E schematically illustrates an example of a Right R earpiece 303a having an attached ear tip 323 in a compressed state 339 (also referred to herein as a first state). The body 337 of the ear-tip 323 is compressed e.g. by the user 101 as tight as possible around the semi-rigid sound bore 335 thereby allowing the user 101 to insert the earpiece 303a into the right ear. Further illustrated is Tx microphone herein in the form of a VPU 317 as disclosed herein.

Fig. 3F schematically illustrates an example of a Right R earpiece 303a being inserted into the ear canal of a user's 101 right ear and having an attached ear tip 323 in a predefined less compressed state 341. The ear tip in Fig. 3F may thus have expanded over a period of time from the first compressed state 339 (e.g. see Fig. 3E) into a second less compressed state 341 where at least a part of an exterior of the tip (323, 323a, 323b) touches and engages with the ear canal of the user 101 thereby preventing or at least attenuating sound from the external environment before reaching further into the ear canal. Thus, the body of the ear-tip 337 has now (e.g. in the second less compressed state 341) adapted to the precise geometry of the inner ear canal structure. It is advantageous that the second less compressed state 341 is different from the fully expanded state, as the less compressed state 341 provide a spring effect, whereas the ear tip 323 may be firmly situated in the ear canal providing an efficient acoustic seal to the external environment and additionally provide a tight and firm interface between the earpiece 303a and the soft tissue 343 and bone structure 345 of the user, thereby allowing vibrations such as jaw bone vibrations 347 (caused by the user speaking) to propagate efficiently from the bone structure 345 into the transmit VPU microphone 317 as disclosed herein thereby enabling the in-ear headset 103 to efficiently pick up the voice of the user 101 even in high noise and demanding environments.

The user compressible material (e.g. 323, 323a, 323b) may advantageously have a predetermined expansion rate so that the tip 323 expands from the (first) compressed state 339 at a first point in time (To) to the (second) less compressed state 341 at a second point in time ($T_1$), where a sound attenuation effect of the tip 323, at the second point in time ($T_1$), has reached at predetermined attenuation level, e.g. or preferably so that the sound pressure level of ambient sound in the ear canal of the user 101 between the proximal end 333 and the inner ear is reduced to a level of 50% or less of the ambient sound outside the ear/ear canal.

It is particularly advantageous to utilize a user compressible material being a foam-material with mechanical properties that allows for a relative long expansion time (from $T_0$ to $T_1$), such as being at least 20 second.

However, a too long expansion time is not preferable, as the fitting procedure of the ear tip 323 then would take too long thereby prolonging the time for the foam to fully expand (e.g. into the second less compressed state 341) and provide the necessary hearing protection for use in demanding environments, for example a law enforcement personal sitting in a vehicle mounting the in-ear tactical communication and hearing protecting headset 103 just before being deployed in a riot control operation and potentially exposed to loud fireworks, etc. making rapid and full hearing protection extremely important. Additionally, as the ear tip 323 is required to work as part of the earpiece 303a for providing both communication and hearing protection capabilities, the ear tip 323 is required to have a hollow core or sound bore 335 for directing an acoustic signal from at least the loudspeaker 319 towards the eardrum of the user. Such an acoustic channel may be of a rigid or simi-rigid nature being less compressible than the surrounding foam material. This design requires the foam material expansion time to be slower than in cases with a standard passive foam earplug that can be squeezed completely together as part of the fitting process.

Accordingly, it is beneficial that the expansion time (from $T_0$ to $T_1$) is between about 20 second to about 100 or about 120 seconds.

In some embodiments, the expansion time (from To to $T_1$) is at least about 30 seconds or about 35 seconds, at least about 40 seconds, at least about 60 seconds, or at least about 70 seconds.

In some further embodiments, the expansion time (from To to $T_1$) is selected from about 20 to about 90 seconds, selected from about 30 to about 90 seconds, selected from about 60 to about 90 seconds, selected from about 70 to about 90 seconds, or selected from about 70 to about 85 seconds.

A typical composition of the foam (e.g. the body 337) may for example be a mixture of Polyurethan foam containing a specific combination of materials that enables the sound isolation characteristics and thermoplastic elastomers a such as a blend of soft absorbing foam with temperature dependent "memory" rubber.

Memory rubber may generally be porous materials composed of a solid polymer skeleton (also called matrix) and air-filled pores. They can be separated into two main groups according to the nature of their polymer skeleton: thermoplastic and thermoset foams. Within these groups, they can even be further differentiated according to their composition, cellular morphology, and other physical and thermal aspects. Their main features are resilience, lightweight, high porosity, and good energy absorption.

Slower expansion (especially in room temperature) will allow the user more time to fit the earpiece properly, which provide advantages such as enabling deeper insertion of the earpiece into the ear canal of the wearer. Generally, the quality of the user's transmitted voice signal will increase with a deeper insertion of the ear tip 323 into the ear. This is because the earpiece 303a utilizes the vibration-based sensor 317 (accelerometer, VPU) to "pick up" the voice signal through sound propagation via vibrations 347 of/in the user's body (e.g. bone structure 345) as they speak. As such, with a larger surface area between the ear tip 323 and the ear canal, the vibrations will have a better signal path to propagate through, into the earpiece 303a, thereby increasing the amplitude/loudness of the transmitted signal. Additionally, deeper insertion may provide better passive hearing protecting (i.e. improve the SNR rating). The degree of passive hearing protection of the ear tip 323 will increase with a deeper insertion of the ear tip 323 into the ear canal as it allows for a longer ear tip 323. The ear tip 323 essentially acts as an absorber/dampener of the external sound that is exposed to the user's ears from the external environment, by which the incoming sounds will be reduced in amplitude significantly compared to free air. By having more foam material between the eardrum and the external sounds, the external signal (noise) will have a longer path through the ear tip before it reaches the eardrum, resulting in a reduced sound pressure level of the noise. Moreover, a deeper insertion may result in a firmer and more secure fit of the earpiece 303a in the ear canal, so the earpiece does not get loose/pull out easily. Additionally, the force required to pull the ear tip 323 out of the user's ear will increase the further the ear tip is inserted into the ear canal since the area applying the friction will be higher when more of the tip 323 are in contact with the ear canal. This is important, as a loose fit could jeopardize users' hearing if an earpiece 303a or 303b comes loose during operation in high noise demanding environments.

A slower foam expansion time, preferrable additionally temperature dependent, may enable the user to make quick refitting of the earpiece easier. In case the user is required to remove one or both the earpieces 303a/303b out of the ears momentarily or for some other purposes, the ear tips 323 (e.g. 323a and 323b) will typically have reached a temperature close to the body temperature of the user (e.g. increased temperature may cause an increased faster expansion. When the earpiece is removed, the temperature of the tip 323 will decrease which may cause the ear tips' expansion time to decrease so that the ear tips 323 may maintain their shape for a little while thereby making quick refitting possible. With foam tips generally known in the field, a quick reinsertion would be quite difficult due to a generally fast expansion rate of standard foam materials (i.e. short expansion time) both at room temperature and at body temperature thus resulting in a rapid shape deformation of the tip upon removal from the ear, which would make quick direct re-insertion difficult. A longer expansion time enables the shape of the foam tip 323 to be maintained for a longer period of time making it easier to refit them quickly and securely.

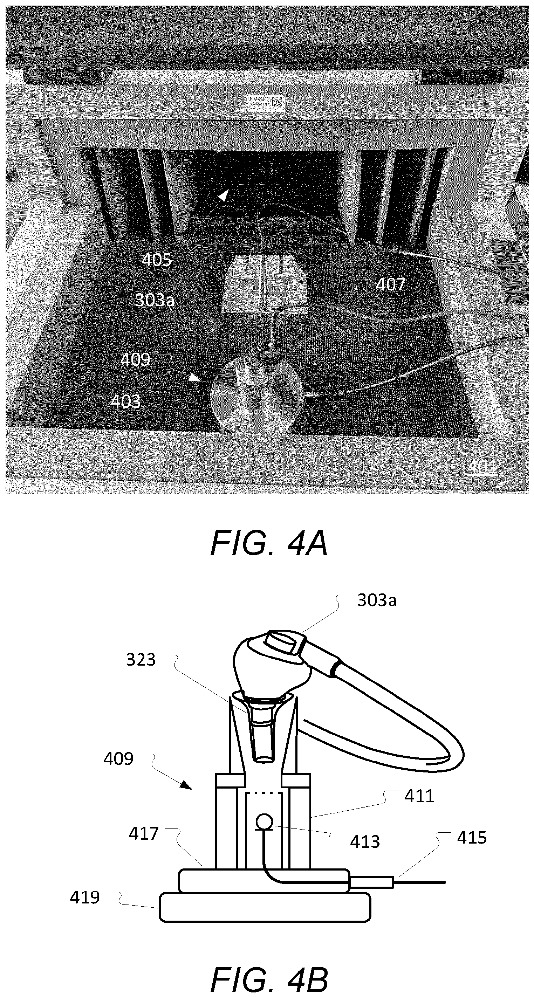

As used herein, the foam expansion time is defined as the time it requires a foam-type ear tip 323 to expand from a first compressed state 339 (e.g. see fig. 3E) to a second less compressed state 341 (e.g. see fig. 3F) when inserted into an ear canal of a user (of any typical user) that results in a 50% of maximum steady state attenuation of the external sound pressure. The foam tip expansion time measurements may be quantified using a standardized test setup 401 as shown in Fig. 4A. The test setup 401 is designed to simulate a realistic user scenario such that the expansion time measured in the test setup 401 may be directly related to real usage of the in-ear device (103, 303a, 303b). Test setup 401 shown in Fig. 4A comprise a sound isolated box 401 that may acoustically block external noises. The box 401 is equipped with a loudspeaker 405 configured to apply a high noise calibrated pink noise signal at a volume of 94 dB SPL (Sound Pressure Level) (mimicking ambient noise of a demanding high-noise environment) and with a frequency range of 100 Hz - 16 kHz. A reference microphone 407 is used to monitor the sound pressure of the applied high noise signal inside the box during the measurement. A stainless-steel acoustic coupler unit 409 is used to simulate the ear canal of the user having a top part being cylindrically shaped with a conical bore adapted to receive the right R 303a or left L 303b in-ear device containing a foam-type ear tip 323. In the bottom of the stainless-steel acoustic coupler unit 409, an internal microphone (see e.g. 411 in Fig. 4B) is placed for measuring the sound pressure inside the unit 409.

Figure 4B schematically illustrate a cross-sectional view of the stainless-steel acoustic coupler unit 409 of Fig. 4A having a right R 303a in-ear device inserted with a compressed foam-type ear tip 323. The stainless-steel acoustic coupler unit 409 used in the test setup may e.g. be a GRAS 43AC Ear Simulator Kit According to IEC 60318-4 comprising a GRAS RA0401 high-frequency ear simulator 411 having a frequency range of 10kHz to 20 kHz, a GRAS 40AG 1/2" Pressure Microphone 413, and a GRAS 26AC 1/4" Preamplifier 415 mounted in a test jig 417 commercially available via the GRAS® Sound & Vibration webpage where the stainless-steel acoustic coupler unit 409 may be placed on a heater 419 for elevating the temperature to a desired level. The loudspeaker 405, the reference microphone 407 and the internal microphone 411 are all connected to a control PC (not shown) for controlling the units 405,407,411 and performing data collection.

An experimental sequence conducted using the test setup 401 for measuring expansion time of the foam-type ear tip 323 according to the invention is performed according to the following: The earpiece 303a with the attached ear-tip 323 in a fully compressed state is inserted into the stainless-steel metal coupler unit 409 and exposed to external noise outside of the coupler unit 409. The sound pressure level (SPL) is then measured inside the coupler unit 409 continuously as a function of time. As the ear-tip 323 expands, the SPL measured by the internal microphone 413 in the coupler unit 409 will decrease until reaching a steady state maximum attenuation value. Before the experiment is initiated, the stainless-steel coupler unit 409 is heated to a temperature of 34°C to simulate a temperature comparable to the human ear. And both the reference- 407 and internal microphone 413 is calibrated and checked to perform equal reading when exposed to external noise (e.g. pink noise signal at 94 dB SPL) via the speaker unit 405. A stepwise description of the experimental sequence method follows:

1. Completely compress the foam-type ear tip 323 (in the first compressed state) around the inner sound bore using the thumb and index finger to squeeze.
2. Insert the earpiece 303a containing the compressed ear tip into the coupler unit 409. At this point, the ear tip 323 should still be compressed and sit very loosely in the coupler.
3. Close the sound box 403 and initiate measurement by starting the external noise exposure and measure the dB SPL every 1-5 seconds using the internal microphone 413. Measurement lasts 180 seconds to ensure that full attenuation is achieved no matter the compression.

The above steps are carried out in immediate extension of each other, such that the starting point of the measurement (To) is not substantially delayed after the placement of the compressed ear tip 303 in the test setup 401 (e.g. the configuration displayed in Figs. 4A and 4B). The experimental sequence is repeated multiple times for the in-ear tactical hearing protection headset 103 according to the invention and multiple times for the prior art headset INVISIO X5.

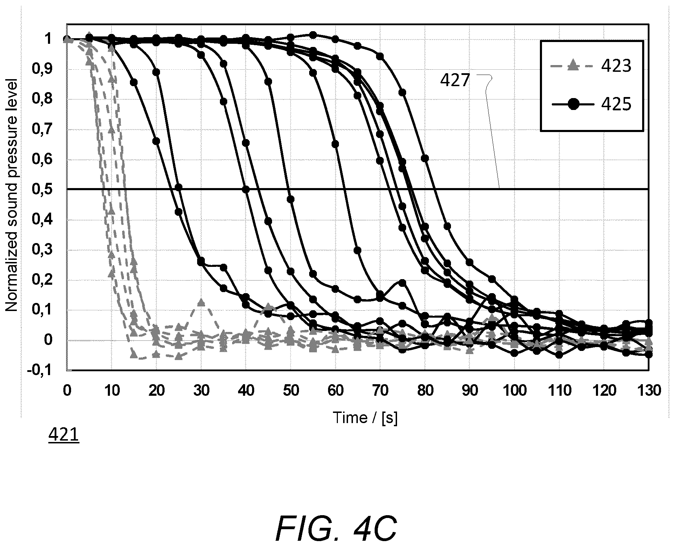

Fig 4C Illustrates a graphical representation 421 of a number of data series showing the expansion time measurements for the foam-type ear tip 323 according to the invention plotted together with expansion measurements of prior art examples, all obtained according to the aforementioned method and test setup 401.

The graphical representation 421 shows the min-max normalized sound pressure level along the y-axis and the time in seconds along the x-axis. The data series 423 indicated by a dark grey dashed line and triangular markers represent data points measured for prior art headsets and the data series 425 indicated by the solid black line and circular markers represent the measurements obtained for the foam-type ear tip 323 of the in-ear hearing protection and hearing protection headset according to the invention and as disclosed herein. The horizontal solid line 427 represents the threshold boundary at 50% attenuation effect relative to the steady state maximum attenuation (i.e. representing the second less compressed stage 341). The expansion time is measured from the time of earpiece insertion (To) in the coupler 409 until a time point ($T_1$) defined by a drop in normalized sound pressure signal to 50% as measured with the internal microphone 413 of the stainless-steel acoustic coupler unit 409.

Real-time Voice filtering using machine learning

Conventional headsets often struggle to deliver fully clear speech quality in noisy and demanding environments. The in-ear tactical communication and hearing protection headset as disclosed herein (see e.g. 103 in Fig. 3A and elsewhere) utilize a vibration-based Voice Pickup Unit (VPU) as a Tx microphone (see e.g. 317 in Fig. 3C) to capture the users voice for transmission via one or more connected communication devices (see e.g. 107,109, 111 in Fig. 1). While a vibration-based Tx microphone 317 may significantly reduce ambient noise compared to traditional air conduction microphones, they may still be sensitive and e.g. pick up internal noises generated from within the user such as chewing, breathing, or other involuntary body sounds, which all can interfere with voice clarity. Existing noise reduction techniques typically rely on basic filtering methods, which may not adequately differentiate between speech and noise in a vibration-based signal response. Additionally, some external factors such as scratching noise from cable routing combined with user movement and/or wind-induced vibrations may lead to a degradation of the voice signal when the in-ear tactical communication and hearing protecting headset 103 is used in demanding environments. To overcome at least some of the above mentioned drawbacks, one or more processors of the in-ear tactical communication and hearing protection system 301 (e.g. see Fig. 3A) may be configured to perform a machine learning based voice signal filtration method with a speech enhancement scheme of the Tx microphone 317 signal to provide a clear voice signal in demanding environments.

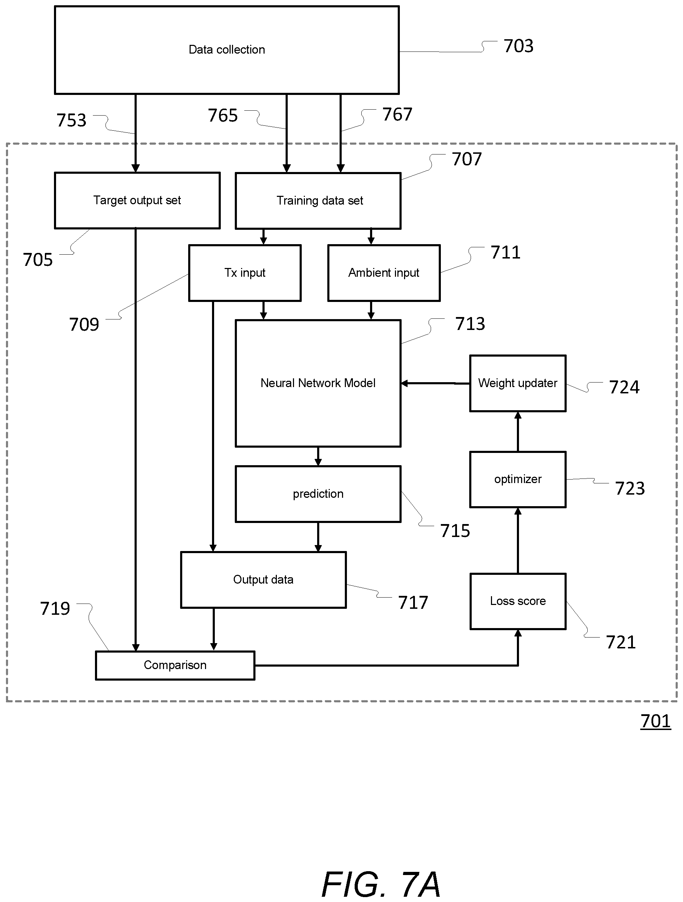

Fig. 5 schematically illustrate an example of a processing architecture 501 of the in-ear tactical communication and hearing protecting system as disclosed herein(see e.g. 301 in Fig. 3A and elsewhere) configured to remove noise and enhance the speech signal quality of the user (see e.g. 101 elsewhere). Illustrated is a real-time voice signal filtration method with a speech enhancement scheme applied using a machine learning engine 515 configured to be operated or executed by one or more processors (i.e. the DSP 207 and/or MCU 205 processors of Fig. 2A) of the PTT control unit (see e.g. 105 elsewhere). The machine learning engine 515 may be designed to process and enhance speech signals in real-time for subsequent transmission via one or more connected communication devices (see e.g. Fig. 1). The machine learning engine 515 may be based on a deep neural network (DNN) model trained using a supervised learning approach as discussed elsewhere in relation to Fig. 7A.

The machine learning engine 515 may be configured to process either the audio signals from the Tx microphone 517 in a single earpiece, such as the Right earpiece 303a or the Left earpiece 303b, or the combined audio signals from the Tx microphone in 317 of both earpieces 303a and 303b in combination. Additionally, the machine learning engine 515 may be configured to process both the audio signals from the Tx microphone 317 and the ambient microphone 315 from a single earpiece, such as the Right earpiece 303a or the Left earpiece 303b, or the combined audio signals from the Tx microphone 517 and the ambient microphone in 315 of both earpieces 303a and 303b in combination. It may be advantageous to configure the neural network engine 515 to process as input both the audio signal from the Tx microphone 317 and the ambient microphone 315 in combination.

A drawback of using a bone conducted speech signal obtained by the Tx microphone 317 may be a relatively limited frequency bandwidth in the vibration-based voice signal propagating through the bone structure (e.g. 347 in Fig. 3F). Traditional air conduction speech signals may comprise a much broader frequency bandwidth providing a better representation of the voice signal. Accordingly, some valuable speech information for providing clear communication may be absent or missing in the bone conducted speech signal if directly transmitted via a radio in a raw form. However, in high noise environments, the bone conducted speech signal is far superior in terms of isolating the users voice as compared to an air conduction microphone, as the bone conducted signal is less impacted by external ambient air borne noise. By combining both a bone conducted speech signal (e.g. recorded by the Tx microphone 317a, 317b) and a traditional air conducted speech signal (e.g. recorded by the ambient microphone 315a, 315b) in a machine learning based filtering process, a superior quality of a voice signal (to be transmitted via a radio) may be achieved, even in demanding environments across a varying and unpredictable ambient noise environment.

As illustrated in Fig. 5, an audio signal 503a from the Tx microphone 317a and an audio signal 505a from the ambient microphone 315a in the Right earpiece 303a is routed into a multiplexing unit 509. Additionally, the audio signal 503b from the Tx microphone 317b and the audio signal 505b from ambient microphone 315b in the Left earpiece 303b is similar routed into the multiplexing unit 509 such that the input Tx microphone audio signal 511 and input ambient microphone audio signal 513 for the neural network engine 515 may be dynamically configured to originate from either of the earpieces (i.e. R 303a or L 303b), both earpieces collectively (i.e. R 303a and L 303b), or any combination thereof. However, it may be advantageous to use the audio signals from a single earpiece only due to resource constrains on the processors (e.g. see e.g. 205, 207 in Fig. 2A) and to decrease power consumption of the PTT control unit 105 which is essential for long operation in demanding environments. Additionally, it may be advantageous to only use the audio signals from a single earpiece to avoid crosstalk.

As previously mentioned, crosstalk is generally understood as an undesirable leakage of an electromagnetic signal from one circuit or channel to another. In radio communication systems, cross-channel signal leakage may cause audio signals from one communication channel to unintentionally "leak" into another communication channel.

In an exemplary scenario, a user 101 being a commander of a team may wear the in-ear tactical hearing protection and communication system 301 connected to two individual radios (see e.g. Fig. 1), one being a secure radio for classified information sharing and the other radio being a team radio. In one situation, the commander may receive a classified voice message (Rx signal) via the secure radio being outputted via the Left 319b and Right 319a speaker units in the headset 103. At the same time, the commander may key the team radio via a PTT button on the PTT control unit 105, thereby activating the Tx microphone 317 and initiating radio transmission to the team members. Thus, the TX microphone 317 may record it the vibrations from the emitting speaker units 319a, 319b which may cause the classified Rx signal from the secure radio to be re-transmitted (Tx signal) via the team radio, thereby allowing unauthorized personnel to hear sensitive or classified information that is transmitted on a different radio channel. Thus, in military operations crosstalk may cause personnel on a non-secure or lower-security channel to unintentionally receive sensitive information from a secure channel. For example, a unit discussing routine logistics might inadvertently hear classified information about strategic movements or intelligence thereby posing a major security risk.

Cross-channel signal leakage may be avoided by configuring the in-ear hearing protecting and communication system such that only one Tx microphone 317a (e.g. in the Right earpiece 303a) is active/used for obtaining the voice of the user while simultaneously focusing any received audio signals from connected communication devices only to be outputted to the user temporarily via a single speaker unit 319b (e.g. in the Left earpiece 303b) opposite of each other, when a radio is keyed by the user (i.e. a PTT button or VOX is activated). Additionally, or alternatively the machine learning based voice signal filtration method with a speech enhancement scheme may be adapted to perform crosstalk cancellation. The signal strength or level of a crosstalk signal may be much lower in intensity than a speech signal from a user, which causes the machine learning engine 515 to remove crosstalk signals as they may be treated as noise.

As seen from Fig. 5, the input audio signal 511 from the Tx microphone(s) 317 may be split into two data streams, where one data stream is forwarded directly to a final noise filtration step 523 and the other data stream is provided as an input component to the machine learning engine 515 for processing as disclosed herein. The input audio signal from the ambient microphone 513 may additionally be split into two data streams, where one data stream is provided as an additional input component to the machine learning engine 515 and the other data stream is forwarded to an output postprocessing operation 519 as explained further in the following. Thus, when suitably trained, the machine learning engine 515 may be configured to receive the input audio signal from the Tx microphone(s) 511 and the ambient microphone(s) 513 as input and output data representing a correction function 517 that, when applied to the input Tx microphone signal 511, filter unwanted or degrading noise from the Tx microphone(s) signal 511 in the noise filtration operation 523 thereby providing a clear/clearer speech signal 525. The output correction function 517 outputted by the machine learning engine 515 may advantageously be adjusted based on the input audio signal 513 from the ambient microphone(s) in an output postprocessing step 519 before the adjusted correction function 521 is applied to the input audio signal 511 from the Tx microphone(s) 317 in the noise filtration step 523. Subsequent to the noise filtration step 523, a final corrected voice signal 525 may be provided for transmission (Tx) via one or more connected communication devices (see e.g. 107,109,111 in Fig. 1). The corrected voice signal 525 may additionally be routed back as an input signal 507a, 507b to one or both loudspeaker(s) 319a, 319b as a so called "sidetone" signal, which make real-time processing important as the voice signal is provided to the users' ears while they are talking making a time delay above 100-150ms unacceptable. Preferably it should be below 50-100ms.

As previously mentioned, a primary purpose of the ambient microphone 315 may be to act as the artificial hearing of the user 101 when wearing the headset 103. The ambient microphones 315a, 315b obtain sound from the surroundings of the user 101 that then can be transmitted to the user's ears via the speaker units 319a, 319b. Thus, the audio signal 505a from ambient microphone 315a in the Right earpiece 303a may be routed via the PTT control unit 105 to input signal 507a for the Right loudspeaker 319a. Additionally, the audio signal 505b from ambient microphone 315b in the Left earpiece 303b may similar be routed via the PTT control unit 105 to be an input signal 507b for the Left loudspeaker 319b, such that the user can hear the surroundings thereby provide situational awareness even though the respective audio connection is not shown in Fig. 5.

Alternatively or additionally, the in-ear communication and hearing protection system 301 may be adapted to perform audio processing of the ambient microphone signals 505a, 505b prior to being routed back to be respective speaker units 319a, 319b of the earpieces 303a, 303b using one or more additional trained neural network model (not shown in Fig. 5) configured to provide active hearing protection and/or enhanced situational awareness by performing real-time processing to filter away unwanted noise contributions, such as wind noise not mechanically removed by the filters 311 and/or perform noise filtration of the input signals 507a, 507b below a harmful limit when played to the user via the speaker units 319a, 319b hereby providing an enhanced situational awareness, where specific segments of interest (SOI) or features in the surrounding audio signal may appear more clear or isolated, such that the user can perform quick vital actions in response. Examples may be enhanced identification of distant gunfire, footsteps, or a person shouting warnings like "Granade incoming", "medic", etc.

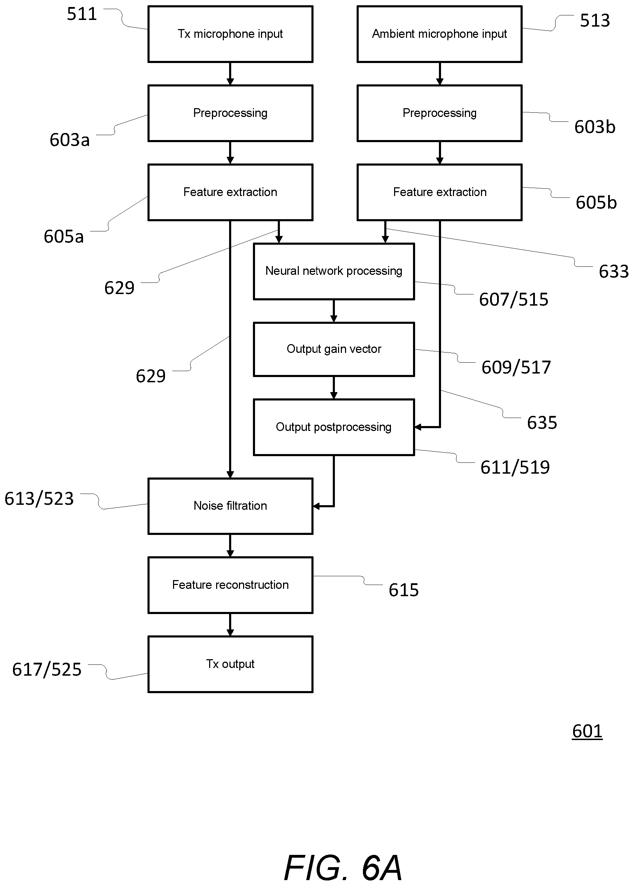

Fig. 6A schematically illustrate an example of steps which may be comprised in a processing method 601 executed by the in-ear tactical communication and hearing protecting system 301 to provide clear and undistorted voice signal via a communication device (see e.g. 107, 109, 111 in Fig. 1A) in demanding environments. One or more processors (see e.g. 205, 207 in Fig. 2A) in a PTT control unit (see e.g. 105 elsewhere) may be configured to execute the method 601 illustrated in fig. 6A-G in accordance with the processing architecture 501 displayed in Fig. 5.

Initially, both a Tx microphone input signal 511 and an ambient microphone input signal 513, e.g. in the form of a continuous voice signal, may be obtained from the one or more Tx microphone(s) 317 and ambient microphone(s) 315 in the R 303a and/or L 303b earpieces as descried previously. The input signals 511, 513 may be obtained in response to the user performing an action, such as pressing and holding a PTT button or similar (see e.g. 223a-d in Fig. 2A and elsewhere) on the PTT control unit, thereby activating the one or more Tx microphones 317 and simultaneously generating a COS/COR signal to key a connected communication device (see e.g. 107, 109, 111 elsewhere) to initiate a voice message radio transmission. Alternatively, a VOX function may activate the generation of the input signals 511,513 in response to the user starts speaking. The Tx microphone input 511 signal and the ambient microphone input 513 signal may be treated in a preprocessing step 603a, 603b followed by a feature extraction step 605a, 605b for preparing the data for processing by the machine learning engine 607/515. The preprocessing steps 603a, 603b and feature extraction steps 605a, 605b may employ at least partly identical operations for the two different input signals from the Tx microphone(s) 511 and the ambient microphone(s) 513. Additionally, the pre-processing-603a, 603b and feature extraction step 605a, 605b may be combined into one step including at least two operations for each input signal 511, 513 separately (i.e. for the Tx microphone input 511 and for the ambient microphone input 513). Speech signals in general are considered to be highly nonstationary signals. However, a speech signal over a short time period of about 10 to 100ms has a characteristic which may be fairly stationary. The preprocessing step 603a, 603b may constitute data treatment operations related to short-time processing techniques in which short segments of audio data in the continuous signal is isolated and processed separately as though they were short segments from a sustained audio with fixed properties. The preprocessing step may thus continuously segment the input audio signals 511, 513 into data frames of about 10-50ms. The feature extraction step 605a, 605b may be a processing sequence for analysing and extracting relevant information/data from the input signals 511, 513, which the neural network processing step 607/515 then may evaluate or use.

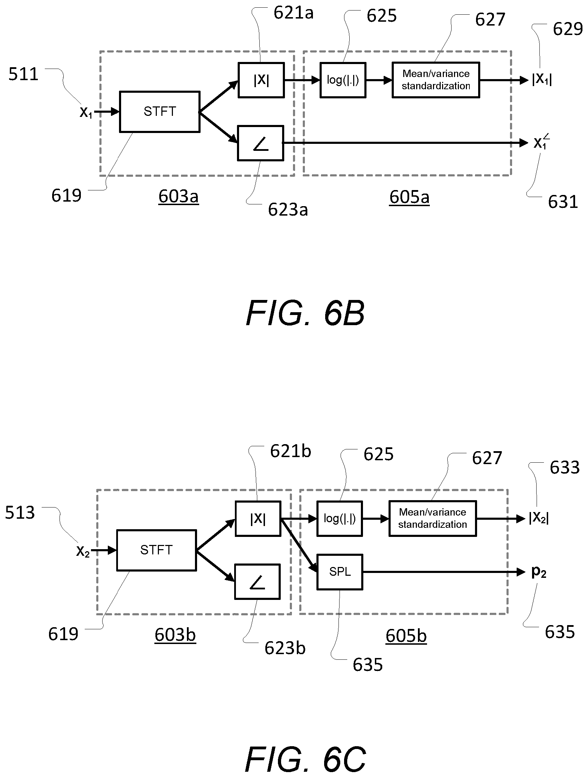

Fig. 6B schematically illustrate an example of a combined pre-processing 603a and feature extraction step 605a for the Tx microphone input signal 511. The operations which may be performed in the combined pre-processing 603a and feature extraction step 605a may generate a spectral representation of the input audio signal 511.

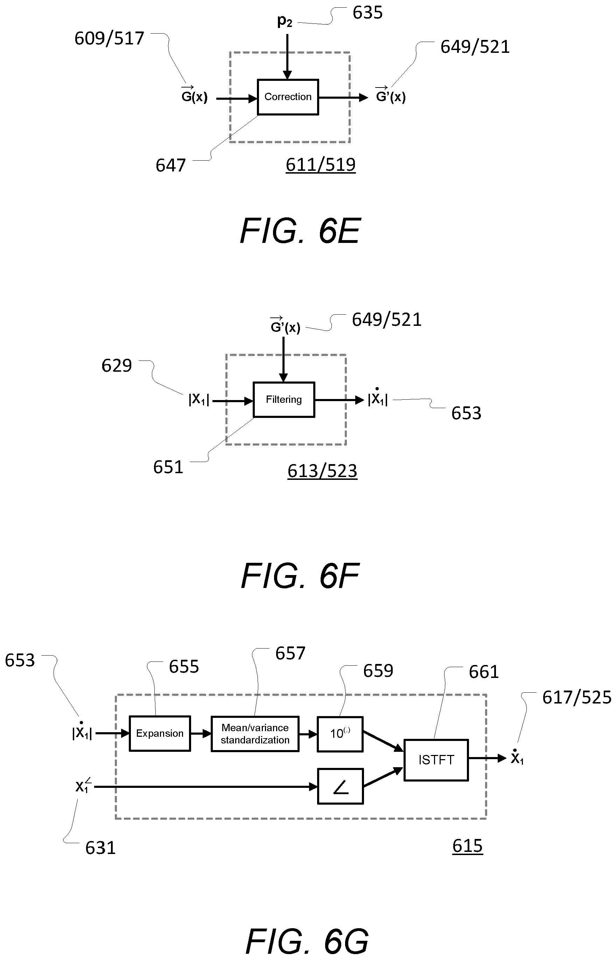

The continuous time varying Tx microphone input signal 511 *"$X_1$"* may be pre-processed into short time audio data frames and transformed from the time domain to the frequency domain in a single operation using a Short Time Fourier Transformation (STFT) 619 algorithm. The STFT operation 619 may alternatively be performed in two steps of initially processing the continuous time varying Tx microphone input signal 511 "Xl" into short time data frames and perform a standard Fourier transformation (FT) analysis subsequently. A Fast Fourier Transform (FFT) size parameter that defines how many frequency bands or "bins" will be applied in the operation 619 (i.e. frequency resolution) may be set to between 32-2048N (bins). A higher FFT Size (i.e. number of bins) will result in a higher frequency resolution of the voice characteristics but would require a longer time span of the data frames, in addition to increased computational time and power usage. For real-time voice analysis of a communication system as disclosed herein, an optimum has been found to be between 64-128N (bins). The output of the STFT spectral transformation 619 may be a complex array which can be split into a magnitude 621a and a phase 623a component. One or both of the magnitude 621a and/or phase 623a component may be used separately or collectively for any subsequent analysis. However, it has been found advantageous to use at least the magnitude component 621 for subsequent data processing according to the method 601. The magnitude component 621a may be subjected to additional mathematical operations such as a logarithm (base-10 or base-2) operation 625 followed by a standardization operation 627 as part of the feature extraction step 605a. The logarithm operation 625 may be advantageous to apply to a voice signal as the human auditory system perceives the strength of the different frequency components similar to a log-scale. The standardization operation 627 of the magnitude component 621 may be performed to transform the data into a Gaussian distribution with zero mean/unit variance to provide a suitable data format for a neural network processing. Thus, the output from the feature extraction step 605a for the Tx microphone input signal 511 "$X_1$" may be in the form of at least a processed Tx magnitude component "$|X_1|$" 629. The processed Tx magnitude component "$|X_1|$" 629 may be provided both as input to the neural network processing step 607/515 and forwarded as an input to the noise filtration step 613/523 as seen in Fig. 6A. Additionally, a phase component "$X_1^{\angle}$" 631 may be outputted from the feature extraction step 605a as an otherwise unprocessed output from the spectral representation operation 619 (i.e. phase component 623a) and may be and passed directly to the feature reconstruction step 615 (see e.g. Fig. 6G).

The continuous time varying ambient microphone input signal 513 may at least partly be treated in a similar way as the Tx microphone input signal 511 in the pre-processing 603b and feature extraction step 605b.

Fig. 6C schematically illustrate an example of a combined pre-processing 603b and feature extraction step 605b for the ambient microphone input signal 513 which contain similar operations as previously described. The output of an identical STFT spectral transformation operation 619 may likewise be a complex array which can be split into a magnitude 621b and a phase 623b component. At least the magnitude component 621b may be subject to additional mathematical operations such as a logarithm (base-10 or base-2) operation 625 followed by a standardization operation 627 as part of the feature extraction step 605b to provide a suitable data format for a neural network processing as explained in relation to Fig. 6B. The magnitude component 621b may additionally be used for a Sound Pressure Level (SPL) analysis where the total sound level in dB (decibel) may be computed as a logarithmic sum of the individual frequency bands (i.e. bins) in one audio data frame. The apparent SPL level may additionally be an average value calculated based on several consecutive audio data frames, such as the previous 50-200 data frames, in order to compute an apparent SPL value 637 for the signal obtained by the ambient microphone(s) 315 on a per second basis rather than in milliseconds to avoid high fluctuations. Thus, the output from the feature extraction step 605b for the ambient microphone input signal 513 "$X_2$" may be in the form of at least a processed Ambient magnitude component "$|X_2|$" 633 and an apparent SPL value 637. The processed Ambient magnitude component "$|X_2|$" 633 may be provided as an additional input variable to the neural network processing step 607/515 and the apparent SPL value 637 may be forwarded to the neural network output postprocessing step 611/519. The generated phase component 623b may be ignored, as the time domain signal for the ambient microphone may not be required to be reconstructed for the specific purpose of the method 601.

The machine learning engine (e.g. see e.g. 515 Fig. 5) may be adapted to perform a neural network processing step 607 as illustrated in Fig. 6A which may be the central part of the processing method 601 for performing the machine learning based voice signal filtration method with a speech enhancement scheme. The neural network processing step 607/515 may utilize one or more trained neural network models to perform a nonlinear mapping between the input and the output features. For the method 601, to enhance the quality of transmitted speech in the headset (see e.g. 103 in Fig. 3A), a regression type algorithm using a supervised learning technique is advantageous as the one or more trained modes should be capable of removing noise (i.e. predicting a clear voice signal) from a noisy voice signal. Several types of neural network (NN) architectures may be used in the neural network processing step 607/515, such as an "artificial neural network" (ANN), like a Feedforward neural network (FNN) or a "convolutional neural network" (CNN), and/or "deep neural network" (DNN) such a Recurrent Neural Network (RNN) or alike. Other types of networks like Generative adversarial networks (GANs) may alternatively be used for audio signal processing. In one example, the machine learning engine 515 may be composed of a plurality of neural network models or components arranged in a consecutive order where an output from one neural network (NN) may be provided as input to another neural network (NN). Alternatively, other non-neural network machine-learning or artificial intelligence architectures or models may be used.

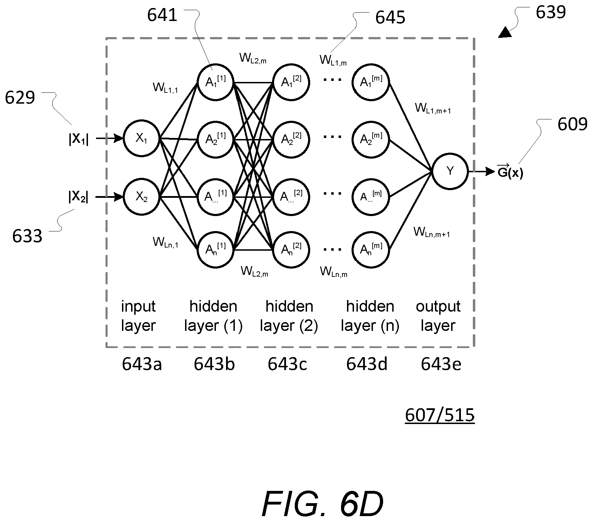

Figure 6D schematically illustrate an example of the neural network processing step 607/515 including a deep neural network (DNN) model 639. The deep neural network (DNN) 639 may be composed of several key components that work together to model the complex relationships in data. The DNN model 639 include a data structure such as an array of neurons 641 which may receive input, apply a transformation, and produce an output. Typically, a neuron 641 performs a weighted sum of the inputs, adds a bias, and applies an activation function. The array of neurons 641 are typically arranged in a plurality of layers 643a-e. The first layer being "input layer" 643a which receives the input data 629, 633. The subsequent layers 643b-d may be referred to as the "hidden layers" and constitute intermediate layers between the input and final "output layer" 643e. These "hidden layers" 643b-d process the inputs 629, 633 through multiple transformations, enabling the network 639 to learn complex patterns and perform predictions. The final layer 643e that produces the network's output 609/517 corresponding to the prediction computed by the DNN model 639. The individual connections between neurons 641 in the network is associated with individual weights 645. Weights 645 are the parameters that the network adjusts and "learns" during a training procedure. The weights 645 determine the balance between the different neurons 641 in the different layers 643a-e and thereby how they contribute to the output. Adjusting the weights allows the model to minimize error and make accurate predictions which is tuned during the training procedure. When the neural network model 639 is trained to a particular level, the weights 645 are static and may not be subject to change. The size of the network i.e. number of neurons 641 and hidden layers 643b-c may vary, however it may be advantageous to limit the size of the neural network 639 as the neural network processing method 607/515 may be configured to be executed on one or more processors (e.g. MCU 205 and DSP 207 in Fig. 2A and elsewhere) on an edge device or similar (e.g. such as a PTT control unit 105 configured to be worn by a person or correspondingly in an in-ear device) with limited computational resources and power constraints. For e.g. optimal performance (and providing real-time processing) of the neural network model, it may be advantageous to utilize more the 6.000 individual weights 645 (i.e. and associated neurons 641 and layers 643b-c).

Both the processed Tx magnitude component "$|X_1|$" 629 and the processed Ambient magnitude component "$|X_2|$" 633 may be provided as input to the deep neural network 639 in the neural network processing step 607/515. The neural network may thus process the input signals 629, 633 and generate an output vector 609/517 in response thereto. The DNN model 639 may be configured such that the output vector may be in the form of a gain vector "$\vec{G}(x)$" 609/517, containing an array of gain value coefficients for each frequency band (i.e. "bins") associated with the processed Tx magnitude component "$|X_1|$".

In alternative embodiments, the DNN model 639 may be configured to output the corrected input signal directly.

However, it is advantageous to configure the DNN 639 to output only a correction function (i.e. gain vector "$\overline{G}(x)$" 609/517), which contain the predicted adjustments to the individual frequency bands (i.e. bins) of the input processed Tx magnitude component 631 in order to obtain a clear or at least clearer voice signal. The computation of a correction function may be computed faster and with less computational effort and power usage compared to a full signal, such that real-time processing of the voice signal may be performed by the in-ear tactical communication and hearing protecting system (e.g. see e.g. 301 in Fig. 3A and elsewhere). The deep neural network 639 may be trained on a large dataset of speech and noise samples, such that the output gain vector 609/517 may be optimized to maximize speech clarity while minimizing noise.

The output gain vector 609/517 may be subjected to a subsequent postprocessing step 611/519. Such a postprocessing step 611/519 may be advantageous to perform in an in-ear tactical communication and hearing protection system (see e.g. 301 in Fig. 3A) as the one or more processors (see e.g. 205, 207 elsewhere) configured to perform the method 601 may be operating on an edge device, configured to be worn by a person, with limited processing capacity, power restrictions, and requirement of processing audio date in real-time. Such constraints may prompt the deep neural network 639 to be optimized for power efficiency and to be able to perform real-time processing of speech signals, making a small neural network size advantageous (i.e. reduced number of neurons 641 and/or hidden layers 643b-d). The drawback of utilizing a small network may be a reduction in performance with respect to signal quality such as speech intelligibility as the mapping and prediction may not be sophisticated enough to cover a large noise input domain (i.e. to provide sufficiently accurate predictions). To address this, the postprocessing step 611/519 may be performed to tune and adjust the output 609/517 from the neural network processing 607/515 so otherwise suboptimal neural network performance (due to performance constrains) may be corrected and/or avoided.

Fig. 6E schematically illustrates one exemplary embodiment of the output postprocessing step 611/519. The output postprocessing step 611/519 may perform a correction operation 647 implementing a mathematical operation defining overall constraints on neural network output 609/517 based on one or more criteria. The neural network output 609/517 may be in the form of a gain vector "$\overline{G}(x)$" 609/517 as previously mentioned, which provide a frequency dependent correction function such as an array containing amplification- or attenuation coefficients in accordance with each individual frequency band (i.e. "bin") of the FFT, STFT, etc. processed Tx microphone input signal 629. The correction operation 647 of the output postprocessing 611/519 may be configured to adjust the value of the individual amplification/attenuation coefficients of the gain vector "$\vec{G}(x)$" 609/517 if they are above or below a threshold value depending on a background noise level ($p_2$) 635 measured by the ambient microphone(s) as explained elsewhere (e.g. in relation to step 603b and step 605b see e.g. Figs. 6A and 6C). The correction operation 647 may be in the form of "clipping function" that limit the value of the amplification/attenuation coefficients of the gain vector to a specified maximum and/or minimum threshold, such that if any amplification/attenuation coefficient exceed the threshold, the value of the coefficient is clipped or truncated to the threshold value. The threshold of the clipping function may be determined as a function of the background noise level (pz) 635, e.g. such as being inversely proportional to the background noise level (pz) 635. This means that if the user is exposed to high background noise while talking (i.e. high SPL value ($p_2$) 365 as measured by the ambient microphone 315) the threshold value may be set low thereby providing a strong constrain or limitation on the gain vector "$\vec{G}(x)$" 609/517. Oppositely, if the user is speaking in low noise situation, the threshold value may be set high effectively providing little or no alteration of the gain vector "$\vec{G}(x)$" 609/517. The correlation between the clipping threshold values and the background noise (i.e. SPL value (pz) 635) may be a continuous non-linear relationship or a step-curve providing different threshold values for SPL value intervals. The output of the correction operation 647 may thus be an adjusted gain vector "$\vec{G}'(x)$" 649/521 containing amplification/attenuation coefficients which may have been modified according to the clipping value determined as a function of the the apparent background noise level (i.e. SPL value (pz) 635).

It is advantageous to apply the output postprocessing step 611/519 in the method 601 since the trained neural network model 639 may be biased to perform a heavy noise filtration with the collateral effect of removing parts of the superimposed voice signal in extreme noise environments. Thus, by constraining the neural network output 609/517 based on the ambient noise level (e.g. SPL value 365 measured by the ambient microphone 315) the in-ear tactical communication and hearing protection system 301 may provide a clear or at least clearer voice signal even in demanding high noise environments.

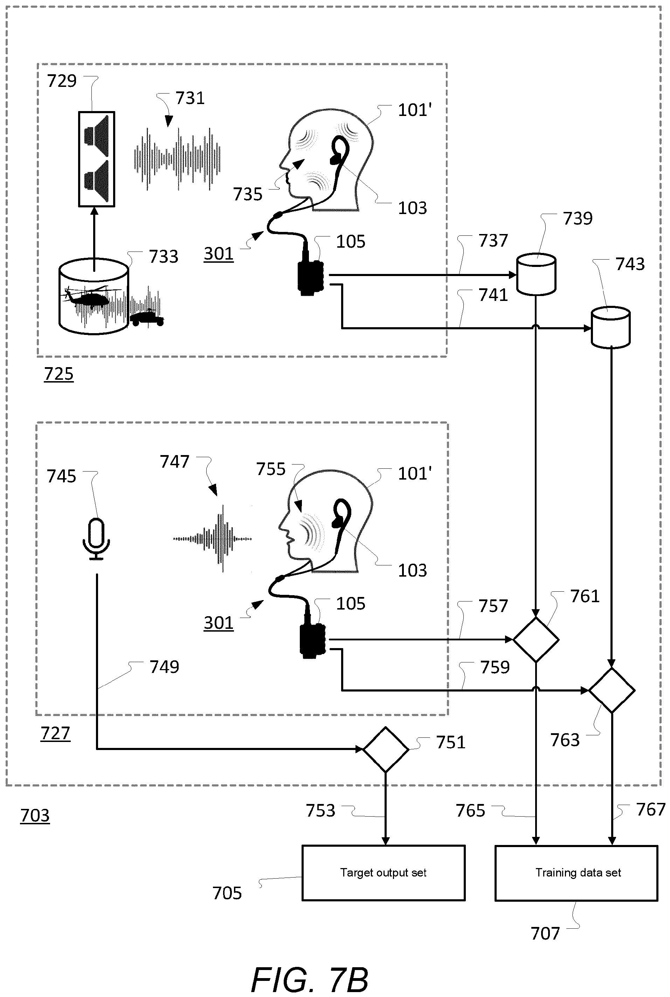

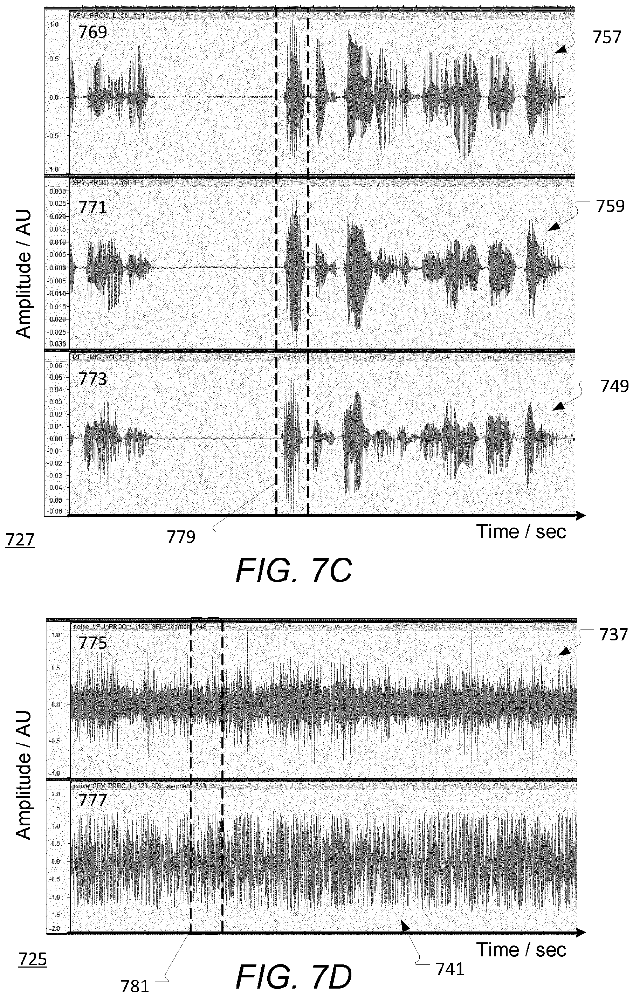

This is because, the neural network processing step 611 may be configured to both remove background ambient noise and enhance the speech signal, as obtained by the Tx microphone 317, simultaneously. This processing may pose an intrinsic challenge for the trained neural network 639 in extreme high noise environments as both the Tx input signal 511 and the ambient input signal 513 may contain a substantial audio signal contribution from the ambient noise. During the training process 701, e.g. or preferably as described in relation to Fig. 7, the neural network model 711 may be configured to adjust one or more of its weights 645 in response to minimizing a loss score 721 or similar based on a comparison 719 between a ground truth clear speech signal 705 and an ambient noise dominated input signal 707 (i.e. in extreme noise environments). Thus, the trained neural network 639 may be biased to perform a too aggressive noise filtration with the collateral effect of removing the superimposed voice signal to minimize the loss function thereby causing a potential degradation of the speech quality in the final Tx output (i.e. without the output postprocessing step 611/519).

The vibration sensitive nature of the Tx microphone 315 is superior in obtaining a speech signal of a user as compared to a normal air conducting microphone (e.g. the ambient microphone 317) in extreme noise environments, and the other way around in low noise environments (ambient microphone 317 is better then Tx microphone 315). Thus, the correction operation 647 may tune or modify the output gain vector 609/517 proposed by the neural network processing step 607/515 depending on the external noise environment. This is advantageous as that the final Tx output 617/525 may then always be better or equal to the unprocessed Tx microphone input 511 (i.e. lower background noise and enhanced speech signal).Said in another way, the correction operation 647 may allow the output gain vector 609/517 to modify the Tx signal 629 in the following step 613/523 more in low noise environments when the ambient microphone input 633 is more reliable than in high noise environments where the ambient microphone input 633 may be too noisy.

The adjusted gain

vector "$\overline{G}'(x)$" 649/521 may then be forwarded to the noise filtration step 613/523 for performing the actual noise filtration of the speech signal 511 obtained by the Tx microphone(s) 317. Fig. 6F schematically illustrates an example of the noise filtration step 613/523 implementing an operation 651 configured to apply the adjusted gain vector "$\overline{G}'(x)$" 649/521 to the processed Tx magnitude component "$|X_1|$" 629. Such an operation may be a standard vector multiplication thereby generating a noise corrected Tx magnitude component "$|\dot{X}_1|$" 653.

A next processing step in the method (see e.g. 601 in Fig. 6A) may be a feature reconstruction step 615 transforming the noise corrected Tx magnitude component "$|\dot{X}_1|$" 653 back into a Tx output 617/525 e.g. or preferably in the form of a continuous time varying audio signal representing a clear or at least clearer speech signal. An example of an implementation of the feature reconstruction step 615 is schematically illustrated in Fig. 6G.

The goal of feature reconstruction step 615 may be to transform the output of the neural network (or e.g. rather or preferably a noise corrected Tx magnitude component version thereof, i.e. the "$|\dot{X}_1|$" 653) back into the same domain as the input to the method 601, in this case, the time domain. A first operation of the feature reconstruction step 615 may be to shift the magnitude values of the distribution for the corrected Tx magnitude component "$|\dot{X}_1|$" 653 back in the original range. This may e.g. be done by scaling the variance by an expansion operation 655 that may be applied to shift the numeric values back to the same natural variance as the target speech signal (e.g. as input Tx signal 511) and subsequently by inverting with a feature standardization operation 657. A next step may be to perform an operation 659 to inverse the previous log10 operation (e.g. see operation 625 in Fig. 6B). A final operation may be to calculate the real and imaginary parts for the Inverse Short Time Fourier transform (ISTFT) 661 using the phase component "$X_1^{\angle}$" 631 (see e.g. also 623a/631 of Fig. 6B) of the original input 511 and the corresponding processed magnitudes. The output "Xl" 617/525 may thus be an enhanced reconstructed version of the Tx microphone input signal 511 as a continuous voice signal in the time domain.

In an alternative embodiment, it may be advantageous to perform an additional processing of the Tx output "$\dot{X}_1$" 617/525 signal before routing the signal to a radio for wireless transmission even though not shown in Fig 6A. The additional processing of the Tx output "Xl" 617/525 signal may be performed by a post adjustment of the Tx output "$\dot{X}_1$" 617/525 signal taking into account the type of the communication device (see e.g. 107,109,111 in Fig. 1 and elsewhere) intended as recipient for the wireless transmission of the Tx output "Xl" 617/525 signal. As previously mentioned, the user 101 may activate a PTT-button 223a-d or similar on the PTT control unit 105 (e.g. see fig 3A) thereby keying a connected radio to transmit a voice signal (i.e. the Tx output 617/525 signal). As the PTT control unit 105 may obtain information related to the communication type (e.g. via cable ship settings or other) a set of specific communication device type instructions may be applied to adjust the Tx output 617/525 signal before transmission via the specific communication device (i.e. keyed radio). Such a specific communication device type adjustment may be advantageous to achieve a clear and undistorted communication as different communication device types may (or may not) apply an intrinsic audio signal processing as part of the internal communication device itself. Such intrinsic audio signal processing algorithms of a radio may in some cases cause an unwanted warping or distortion of the Tx output "Xl" 617/525 signal, if for example an additional speech optimization algorithm, signal compression, analog to digital conversion (VoIP), or vice versa, etc. are applied by the radio device substantially altering the original signal (i.e. altering the Tx output "Xl" 617/525 signal). Thus, the specific communication device type adjustment may be a radio device audio encoding optimization, such that the transmitted signal by the radio may be or remain clear and undistorted despite any communication device type variations.

Neural networks training method

The trained neural network engine 515 configured to perform the neural network processing 607 in the in-ear tactical communication and hearing protecting system as disclosed herein (see e.g. 301 elsewhere) may be trained according to the training method 701, schematically illustrated in Fig. 7A, to provide real-time processing of a user's voice signal to produce at least clearer and at least more undistorted communication in demanding environments. Figure 7A schematically illustrate an initial data collection 703 used to generate a training data set 705 and a target output set (i.e. ground truth) 707 (or rather a series/sufficient plurality of such) for performing the training method 701 following, as an example, a supervised learning scheme.

The data collection 703 may, as an example, be performed in accordance with the process schematically illustrated in Fig. 7B. The data collection 703 may be divided into two segments or modes in order to obtain a high quality and realistic target output 705 and training data set 707. One mode or a first mode being a "mute mode with high noise background" 725 used to obtain noisy response signals from the in-ear tactical communication and hearing protection system 301 and another mode or a second mode being a "speech mode with a silent background" 727 used to obtain clear speech signals from the in-ear tactical communication and hearing protection system 301. In the "mute mode with high noise background" 725, a test subject 101' wearing the in-ear technical communication and hearing protecting system (see e.g. 301 in Figs. 3A and 3B and elsewhere) may be situated in a sound isolated room including one or more external loudspeaker(s) 729 directed towards the test subject 101'. The loudspeaker(s) 729 may be configured to generate a high sound pressure such as between 60-140 dB SPL to simulate both a quiet and a loud real-world environment.

The test subject 101' may be instructed not to speak but otherwise produce a variety of natural so called "involuntary sounds" such as breathing, sighing, swallowing, lip smacking, chewing, teeth grinding, sniffling, etc. while also move around to produce sounds from clothes, worn/carried equipment, and cables, turn the head from side to side, up and down, clicking, tapping, or rustling sounds made by the movement, etc. Simultaneously, the loudspeaker(s) may expose the test subject 101' to a plurality of loud airborne noise segments 731 to simulate different demanding environment situations. Such noise segments 731 may be obtained from a database 733 containing an audio data library from demanding environments, such as heavy machine noise, gunshots, helicopters, explosions, etc.

The in-ear tactical communication and hearing protection headset 103 may thus obtain several noise responses such as the vibration-based noise signal response 735 generated when the test subject 101' is exposed to the noise segments 731, "involuntary sounds", and other vibration-based audio artifacts originating from the equipment (e.g. cable scratching and clothing rustle etc) via the Tx microphone(s) (see e.g. 317 in Fig. 3C) and obtain the air borne noise segments 731 directly via the ambient microphone(s) (see e.g. 315 in Fig. 3C). The PTT control unit 105 may be configured to output raw audio Tx signals 737 obtained by Tx microphone(s) thus containing noisy signals originating from the equipment (e.g. cable scratching, etc.), the test subject 101' itself (e.g. involuntary sounds), and the test subject' 101' exposure to demanding environments to a Tx noise signal database 739. Additionally, the PTT control unit 105 may be adapted to output raw noise ambient signals 741 obtained by the ambient microphone(s) in response to the exposure to demanding environments to an ambient noise signal database 743. Preferably, the vibration-based noise signal response 735 and the ambient microphone signal response (and thereby their raw output versions thereof, 737, 741) are obtained at the same time, i.e. they are both obtained of the same noise segment(s) 731 thus offer different ways of obtaining or recording the noise 731 where each way provides its version or its way of obtaining or recording the noise 731, including particularities of each way, respectively. In other words, each way provides a different propagation path (e.g. air borne/vibration-based) and subsequent recording of the noise 731. Herein, obtaining the noise 731 via the Tx microphone(s) is also denoted, as least in some embodiments, obtaining the noise 731 (with no speech) in accordance with a second way. Additionally, obtaining the noise 731 via the ambient microphone(s) is also denoted, as least in some embodiments, obtaining the noise 731 (with no speech) in accordance with a third way.

In the "speech mode with a silent background" 727 (i.e. no noise from any demanding environments), a test subject 101' wearing the in-ear technical communication and hearing protecting system (see e.g. 301 in Figs. 3A, 3B, and elsewhere) may be situated in a sound isolated room including an external microphone 745. The external microphone 745 may be and preferably is a high quality professional stationary voice recording microphone, such as a Shure SM7B, Sennheiser e935, Audio-Technica AT2010, or the like, and is placed in close vicinity of and facing the test subject 101'. The external microphone 745 may be configured to obtain an air conducted voice signal 747 from the test subject 101' when speaking to produce a high quality, clear, and undistorted voice signal 749. Preferably the high quality, clear, and undistorted voice signal 749 may undergo a subsequent filtering step 751 to be processed and optimized for speech pronunciation rather the pure audio quality thereby generating a reference voice signal 753 that is optimized for speech intelligibility rather than audio fidelity when transmitted via a narrow band (e.g. 0-40 kHz) RF wireless transmission via a radio. The filtering step 751 may be configured to apply an equalizer function with an enhancement scheme for selective enhancement of lower frequency bands between 0-3kHz, preferably between 500-1500 kHz, thus optimizing the reference signal for radio transmission purposes. The voice signal 749 obtained by the external microphone 745 (also referred to as obtaining the voice signal in accordance with a first way) may thus be used as part of the target output set 705 constituting a ground truth for the supervised training method 701 illustrated in Fig. 7A. The test subject 101' may be instructed to move around, turn the head from side to side, up and down while reading aloud from a manuscript or other in order to generate both the air conducted voice signal 747 and a bone conducted speech signal 755. The in-ear tactical communication and hearing protection headset 103 may thus obtain the vibration-based speech signal 755, involuntary sounds and movement induced vibrations using the Tx microphone(s) (see e.g. 317 in Fig. 3C), and the air conducted voice signal 747 may simultaneously be obtained by the ambient microphone(s) 315 of the in-ear tactical communication and hearing protecting headset 103. The PTT control unit 105 may be configured to output a raw intermediate Tx signal 757 obtained via the Tx microphone(s) 317 (i.e. obtained in accordance with the second way) containing the vibration-based speech signal 755 and audio artifacts originating from the test subject 101'and equipment (e.g. cable scratching) as previously described (i.e. involuntary sounds) and additionally output a raw intermediate ambient signal 759 obtained via the ambient microphone(s) 315 (i.e. obtained in accordance with the third way) containing the air borne speech signal.

The intermediate Tx signal 757 may subsequently be modified in a processing step 761 by mixing the signal 757 with noise data from a Tx noise signal database 739 thereby generating training Tx signal 765 data. Similarly, the intermediate ambient signal 759 may also be modified in a processing step 763 by mixing the audio signal 759 with noise data from the ambient noise signal database 743 thereby generating a training ambient signal 767 data. Thus, a reference voice signal 753 and the corresponding pair of a training Tx signal 765 and a training ambient signal 767 may constitute one data entity (i.e. a ground truth and a pair of associated training data) of the target output set 705 (i.e. a reference voice signal 753) and the training data set 707 (i.e. a pair of a training Tx signal 765 and a training ambient signal 767). The data set 707 may comprise multiple pairs of training Tx signal/data 765 and training ambient signal/data 767, both with and without noise data.

In summary, obtaining data according to the first way may comprises providing an acoustic signal by a microphone or transducer of a first type being a high quality professional stationary voice recording microphone or transducer (e.g. 745). Obtaining data according to the second way comprises providing an acoustic signal by a microphone or transducer of a second type being a vibration pick-up sensor or vibration sensitive transducer (e.g. 317 Fig. 3C), preferably of an in-ear communication and hearing protection device 103. Obtaining data according to the third way comprises providing an acoustic signal by a microphone or transducer of a third type being an ambient microphone (e.g. 315 in Fig. 3C).

Fig. 7C Illustrates a graphical representation of three exemplary audio signal data in a first subplot 769, a second subplot 771, and a third subplot 773 arranged in a vertical stack, representing training data used to train the neural network model 713 to obtain a trained neural network model 607/515 according to the training method 701. The third subplot 773 in Fig. 7C is showing an example of the first data (e.g. 705, 753) representing a reference speech signal including a speech signal 747 of the test subject 101' obtained in accordance with the first way. The first subplot 769 in Fig. 7C is showing an example of the second data 707, 709, 765 representing a training transmit (Tx) signal including at least the speech signal 747 obtained in accordance with the second way. The second subplot 771 is showing an example the third data 707, 711, 767 representing a training ambient signal including at least the speech signal 747 obtained in accordance with the third way. The subplots 769,771,773 are illustrated in a vertical stack arrangement aligned according to the same x-axis being time, as the first (e.g. 757), second (e.g. 759) and third data (e.g. 749) are respectively obtained at substantially the same time in response to a speech signal 747 of the test subject 101'. Each of the subplots 769,771,773 may have different scales on their respective y-axis, showed in arbitrary units (AU). The y-axis of the individual subplots 769,771,773 may be synchronized so that the data scales relative to each other in a meaningful way, such that changes or trends in the signals can be compared proportionally, even though the signals might have different ranges. The audio signal data represented in the first subplot 769, the second subplot 771, and the third subplot 773 may be obtained according to the second mode being a "speech mode with a silent background" 727 used to obtain clear speech signals from the in-ear tactical communication and hearing protection system 301, as described previously.

In one embodiment, the dashed box 779 in Fig. 7C show an example of a data entity (e.g. target output and training data pair) including a part of the reference voice signals 753 (segment in third subplot 773) being the target output 705 element and a corresponding pair including a part of the training Tx signal 765 (segment in first subplot 769) and a part of the training ambient signal 767 (segment in second subplot 771) being the training data 707 element of a data entity. A signal part may vary in length between 10 ms to 10 s, preferably between 20 ms to 150 ms for real-time performance. The dashed box 779 in Fig. 7C may thus represent an example of a data entity including target output (i.e. ground truth) and corresponding training data, not including noise representative of loud noises of a demanding environment, used to train an artificial intelligence or machine learning model, and may thus advantageously be used for speech optimization purposes.

Fig. 7D Illustrates a graphical representation of two exemplary audio signal data in a fourth subplot 775 and a fifth subplot segment 777 arranged in a vertical stack, representing noise data used to train the neural network model 713 to obtain a trained neural network model 607/515 according to the training method 701. The fourth subplot 775 in Fig. 7D is showing exemplary noise data representing a training transmit (Tx) signal 737 including noise (e.g. 731, 733) being representative of loud noises of a demanding environment, obtained in accordance with the second way. The fifth subplot 777 in fig. FD is showing exemplary noise data representing a training ambient signal 741 including noise (e.g. 731, 733) being representative of loud noises of a demanding environment, obtained in accordance with the third way.

The subplots 775,777 are illustrated in a vertical stack arrangement aligned according to the same x-axis being time, as the noise data 737,741 are obtained at around substantially the same time in response to loud noises 731 of a demanding environment. Both the fourth subplot 775 and the fifth subplot 773 may have different scales on their respective y-axis, showed in arbitrary units (AU). The y-axis of the individual subplots 775,777 may be synchronized so that the data scales relative to each other in a meaningful way, such that changes or trends in the signals can be compared proportionally, even though the signals might have different ranges. The audio signal data represented in the fourth subplot 775 and fifth subplot 777 may be obtained according to the first mode being a "mute mode with high noise background" 725 used to obtain noisy response signals from the in-ear tactical communication and hearing protection system 301, as described previously.

In another embodiment, the dashed box 781 in Fig. 7D show an example of a data entity including a pair of noise data, representative of loud noises of a demanding environment including a part of the noise Tx signal 737 (segment in forth subplot 775) and a part of the noise ambient signal 741 (segment in fifth subplot 777). A signal part may vary in length between 10 ms to 10 sec, preferably between 20 ms to 150 ms for real-time performance. The parts of the noise signals 737,741 as showed in the dashed box 781 in Fig. 7D may be mixed with the speech signal pair of the part of the training Tx signal 765 (segment in first subplot 769) and a part of the training ambient signal 767 (segment in second subplot 771) shown in Fig.7C, such that the second data (e.g. 707, 709, 765), representing a training transmit (Tx) signal including the speech signal (e.g. 747), further includes noise (e.g. 731, 733) being representative of loud noises of a demanding environment, and the third data (e.g. 707, 711, 767), representing a training ambient signal including the speech signal (747), further includes noise (e.g. 731, 733) being representative of loud noises of a demanding environment. Hence, the dashed box 779 in Fig. 7C in addition with the dashed box 781 in Fig 7D may thus represent an example of a data entity including target output (i.e. ground truth) and corresponding training data, including noise representative of loud noises of a demanding environment, used to train the artificial intelligence or machine learning model 713, and may thus advantageously be used for both noise suppression and speech optimization purposes.

Advantageously, both data entities representing training data including noise representative of loud noises of a demanding environment and training data not including noise representative of loud noises of a demanding environment may form part of the data framework (e.g. collection of data entities) used to train the neural network model 713 according to the training method 701. Such that the final Tx output 617/525 of the trained neural network model 607/515 may be optimized for speech intelligibility and noise suppression and thereby by enabled to provide clear and undistorted communication in demanding environments.

Accordingly, at least in some embodiments, a method (701) of training an artificial intelligence or machine learning method or component (515, 607, 713) to be executed by at least one device (103, 303a, 303b, 107, 109, 111, 205) of a communication system is provided, where the artificial intelligence or machine learning method or component (515, 607) is configured to generate real-time processing of a user's voice signal (525) in a demanding environment, the method (701) comprising

> a) obtaining first data (705, 753) representing a reference speech signal including a speech signal (747) of a user (101, 101') obtained in accordance with a first way,
> b) obtaining second data (707, 709, 765) representing a training transmit (Tx) signal including the speech signal (747) obtained in accordance with a second way,
> c) obtaining third data (707, 711, 767) representing a training ambient signal including the speech signal (747) obtained in accordance with a third way,
> d) providing the second data and the third data to the artificial intelligence or machine learning method or component (515, 607, 713) generating a predicted output (715, 717) in response thereto,
> e) comparing (719) the predicted output (715, 717) and the first data (705, 753) and determining (721) a difference therebetween, and
> f) updating parameters (645) of the artificial intelligence or machine learning method or component (515, 607, 713) in response to the determined difference (721, 723, 735),

wherein the method (701) further comprises repeating steps a) - f) for new first, second, and third data a plurality of times, typically a large number of times, until the generated difference of the predicted output (715, 717) and the first data (705, 753) is within a predetermined threshold or the improvement of generated difference (from cycle to cycle) stops improving sufficiently.

Additionally, in some further embodiments,

- the second data (707, 709, 765), representing a training transmit (Tx) signal including the speech signal (747), further includes user-generated noise (e.g. such as natural or involuntary noises), e.g. or preferably obtained in accordance with the second way (757, 761), and/or
- the third data (707, 711, 767), representing a training ambient signal including the speech signal (747), further includes user-generated noise e.g. or preferably obtained in accordance with the third way (759, 763).

Additionally, in some further embodiments,

- the second data (707, 709, 765), representing a training transmit (Tx) signal including the speech signal (747), further includes noise (731, 733) being representative of loud noises of a demanding environment,
- the third data (707, 711, 767), representing a training ambient signal including the speech signal (747), further includes noise (731, 733) being representative of loud noises of a demanding environment, and
- the first data (705, 753), representing a reference speech signal including the speech signal (747), does not include noise being representative of loud noises of a demanding environment.

A comprehensive collection of data entities of respective reference voice signals 753 and corresponding pairs of a respective training Tx signal 765 and a training ambient signal 767 constituting the target output set 705 and the training data set 707, respectively, may be generated by repeating the data collection process 703 using multiple test subjects from multiple nationalities and different genders as well as obtaining ambient-741 and Tx noise data 737 involving a range of different relevant noise environments and combining and mixing audio segments and signal parts from the different signals (e.g. 737, 741, 757, 759).

Before starting the training process 701, the training data set 705 and target data set 707 may be split into a two data parts. One data part (i.e. a verification data pool) for evaluation of the performance of the neural network model 713 after the training process 701 is completed, and one data part for actual training of the neural network model 713.

Referring now to training process illustrated in Fig. 7A. The training Tx signals 765 in the training data set 707 may be processed into a Tx input 709 data having a suitable format for training the neural network model 713, which may be or preferably is similar to the data processing described in relation to Fig. 6B. Similarly, the training ambient signals 767 in the training data set 707 may be processed into an Ambient input data 711 also having a suitable format for training the neural network model 713 e.g. or preferably similar to the data processing described in relation to Fig 6C.

The untrained neural network model 713 architecture and type may be similar to the trained neural network 607/515 of Fig. 6D where the difference is that the untrained neural network model 713 may initially have more or less arbitrary weights 645 and biases assigned (or alternatively set in any other suitable way) to the individual notes 641 when the training process 701 is started or initially assigned. The goal of the training process 701 may be to update the weights 645 and biases of the individual notes in an iterative manner until the prediction 715 of the neural network 713 is very close or basically identical (for all practical purposes) to the target data set 705 (i.e. ground truth).

The training method 701 may be performed in an iterative manner, where each iteration cycle may comprise a forward pass followed by a backwards pass.

During a forward pass, the Tx input 709 and Ambient input 707 is passed through the neural network model 713 layer by layer (see e.g. 643b-d in Fig. 6D) where each layer applies transformations, such as weighted sums and added activation functions. This process results in a prediction 715 as output (see e.g. also 609 in Fig. 6D). The prediction 715 may be forwarded to an output data 717 processing step performed e.g. or preferably similar to the combined noise filtration 613/523 and feature reconstruction step 615 (see e.g. Figs. 6A and 6F) where a duplet of the Tx input signal 709 is combined with the prediction 715 e.g. or preferably according to the processing explained in relation to Fig. 6F and 6G. By separating the prediction 715 and output data 717 processing steps into two individual steps, the training process 701 enables the neural network model 713 to generate the prediction 715 as a correction response (i.e. gain vector) to the Tx input 709 rather than data representing the fully corrected signal, which is advantageous as previously described. The signal from the output data step 717 may subsequently be directed to a comparison step 719. The comparison step 719 may contain a so-called "loss function", which may be configured to compare the output data 717 (i.e. corrected Tx input data) to the corresponding true target value of the Target output set 705 and calculate a loss score 721 as a quantitative value of the difference or "error" between the data sets. Thus, the loss score 721 value indicates how far the model's prediction 715 is from the actual target (i.e. ground truth). For regression tasks, typical loss functions 719 may be the Mean Squared Error (MSE) or Mean Absolute Error (MAE). For example, MSE calculates the squared differences between predicted 717 and the ground truth 705. Designing a proper loss function for audio processing tasks in machine learning is significant as it guides the model during training to focus on relevant aspects of the audio signal (such as noise removal) while preserving and enhancing useful information (i.e. speech signal) and avoiding trivial solutions such as removing all of the audio signal or introducing distortions. Advantageously, effective loss functions for the audio processing may be MSE with weighted frequency bands or a hybrid loss function as a combination of different loss functions.

In the subsequent backwards pass, the error score 721 may be used in the optimizer step 723 where the gradient of the loss function may be computed with respect to each weight (see e.g. 645 in Fig. 6D) of the neural network model 713. The calculation of the gradients for each individual weight 645 may be achieved using a method called backpropagation, which applies the chain rule of calculus. Backpropagation computes the gradient of the loss function 719 for each weight 645 by propagating the error backward through the layers of neural network model 713. The gradient may be calculated as the partial derivative of the loss function 719 with respect to the network's weights 645. Thus, the gradient may represent the rate of change of the loss function 719 when a small perturbation is applied to the weights 645 one by one. The gradients calculated in the optimizer step 723 may thus instruct the neural network model 713 how much and in which direction (positive or negative) a particular weight 645 value of the neural network model 713 should be adjusted to minimize the loss (i.e. loss score 721). The subsequent weight updater step 724 may then apply the gradients and adjust the parameters (i.e. weights and biases) of the neural network model 713 using an optimization algorithm. A common algorithm used for this purpose is e.g. the Gradient Descent (or its variants, like Stochastic Gradient Descent (SGD)).

The training method 701 may perform consecutive cycles of forward- and backward passes to ongoingly train the neural network model 713, gradually reducing the loss function's value as the network optimizes its parameters. The process of making predictions, calculating the loss, performing backpropagation, and updating the weights may be repeated for every data entity of reference voice signal 753 and corresponding pairs of training Tx signal 765 and training ambient signal 767 of the target output set 705 and the training data set 707. The training method may be completed when reaching a point where the loss scores 721 are minimized or sufficiently small indicating that the neural network model has learned the relationship between the input features 707 and the ground truth 705 (sufficiently or adequately for the given purpose). The training method may be stopped when convergence occurs, hence when the loss scores 721 stops decreasing significantly thereby indicating that the neural network model 713 has reached or is close to an optimal set of weights 645. A final step may be a validation step using the verification data pool. The neural model 713 may be evaluated on the verification data pool to check the performance on 'unseen' data (i.e. not part of the training as such). The same comparison step 719 i.e. loss function (MSE, MAE, etc.) may be used to measure how well the model performs (e.g. generalizes). Additional metrics like R-squared (for adequacy of fit), R-MSE, or adjusted R-squared might be used to evaluate the neural model 713. When the training process 701 is completed and validation is successful, the trained neural network model 607/515/713 may be deployed in the in-ear tactical communication and hearing protection system 301 performing the real-time voice processing 601 as shown in fig 6A.

Some preferred embodiments have been shown in the foregoing, but it should be stressed that the invention is not limited to these, but may be embodied in other ways within the subject matter defined in the following claims.

It should be emphasized that the term "comprises/comprising" when used in this specification is taken to specify the presence of stated features, elements, steps or components but does not preclude the presence or addition of one or more other features, elements, steps, components or groups thereof.

In the claims enumerating several features, some or all of these features may be embodied by one and the same element, component or item. The mere fact that certain measures are recited in mutually different dependent claims or described in different embodiments does not indicate that a combination of these measures cannot be used to advantage.

In the claims, any reference signs placed between parentheses shall not be constructed as limiting the claim. The word "comprising" does not exclude the presence of elements or steps other than those listed in a claim. The word "a" or "an" preceding an element does not exclude the presence of a plurality of such elements.

The mere fact that certain measures are recited in mutually different dependent claims does not indicate that a combination of these measures cannot be used to an advantage.

It will be apparent to a person skilled in the art that the various embodiments of the invention as disclosed and/or elements thereof can be combined without departing from the scope of the invention as defined in the claims.

## Claims

1. A communication system configured to be used in a demanding environment, the communication system comprising

   - at least one communication and hearing protection device (103, 303a, 303b), in particular at least one in-ear communication and hearing protection device (103, 303a, 303b), configured to be worn by a user (101), the communication and hearing protection device (103, 303a, 303b) comprising

     ◦ an ambient microphone (315, 315a, 315b) configured to provide an ambient microphone input signal (505a, 505b, 513), and
     ◦ a transmit (Tx) microphone (317, 317a, 317b) configured to provide a transmit microphone input signal (503a, 503b, 511) in response to registering speech from the user (101),

   - one or more processors (205, 207) configured to implement and execute a trained artificial intelligence or machine learning method or component (515, 607) to generate a correction function or signal (517, 609) in response to the ambient microphone input signal (505a, 505b, 513) and the transmit microphone input signal (503a, 503b, 511).

2. The communication system according to claim 1, wherein the correction function or signal (517, 609) enables a corrected or improved voice signal (525, 617), and/or wherein the one or more processors (205, 207) is/are further configured to apply the correction function or signal (517, 609) to the transmit microphone input signal (503a, 503b, 511) thereby providing a corrected or improved voice signal (525, 617).

3. The communication system according to claim 2, wherein the corrected or improved voice signal (525, 617) is provided as signal for transmission, e.g. to be received by one or more communication devices (107, 109, 111), e.g. by one or more one or more radios (109, 111).

4. The communication system according to any one of claims 1 - 3, wherein the transmit (Tx) microphone (317, 317a, 317b) is

   - a vibration-based transducer (317), e.g. an accelerometer type vibration sensitive transducer such as a digital voice pick up (VPU) type microphone, acoustically coupled with the user (101), when the user (101) is wearing the communication and hearing protection device (103, 303a, 303b), where the vibration-based transducer (317) is configured to obtain a voice signal of the user (101) in response to vibrations caused by the user (101) speaking and provide the transmit microphone input signal (503a, 503b, 511) in response thereto.

5. The communication system according to any one of claims 1 - 4, wherein the ambient microphone (315, 315a, 315b) is

   - a microphone configured to register an airborne ambient acoustic signal of a demanding environment and provide the ambient microphone input signal (505a, 505b, 513) in response thereto.

6. The communication system according to any one of claims 1 - 5, wherein

   - the ambient microphone input signal (505a, 505b, 513) represents an airborne acoustic signal, and
   - the transmit microphone input signal (503a, 503b, 511) represents a vibration signal.

7. The communication system according to any one of claims 1 - 6, wherein the generated correction function or signal is or comprises data representing a gain vector or similar, wherein the gain vector or similar comprises a number of predicted adjustment values, predicted by the trained artificial intelligence or machine learning method or component (515, 607), for different segments or parts of the transmit microphone input signal (503a, 503b, 511) or a processed version thereof, where an adjustment value for a particular segment or part specify whether the transmit microphone input signal (503a, 503b, 511) or a processed version thereof in the particular segment or part should be kept or should be increased or decreased and to what extent.

8. The communication system according to claim 7, wherein the predicted adjustment values each are limited or clipped to be within predetermined maximum and/or minimum threshold, wherein the predetermined maximum and/or minimum threshold are set in response to a derived or estimated sound pressure value (637) representing or estimating an external background noise level, wherein the predetermined maximum and/or minimum threshold are set to relatively high threshold(s) in case the derived or estimated sound pressure value (637) indicates no or little background noise and are set to relatively low threshold(s) in case the derived or estimated sound pressure value (637) indicates a relatively high background noise.

9. The communication system) according to any one of claims 1 - 8, wherein the communication system is configured to:

   - apply a processed version of the generated correction function or signal (517, 609) to the transmit microphone input signal (503a, 503b, 511) thereby filtering or reducing noise from the transmit microphone input signal (503a, 503b, 511) and providing the corrected or improved voice signal (525, 617).

10. The communication system according to any one of claims 1 - 9, wherein the transmit (Tx) microphone (317, 317a, 317b) is digital, and wherein the communication system or the communication and hearing protection device (103, 303a, 303b) comprises a dedicated direct digital-to-analog converter (DAC) circuitry (349) coupled to the transmit (Tx) microphone (317, 317a, 317b) and configured to perform lossless front-end digital to analog signal conversion.

11. The communication system according to claim 10, wherein the transmit (Tx) microphone (317, 317a, 317b) is configured to output a digital Pulse Density Modulation (PDM) signal representing an obtained voice signal of the user (101), and wherein the dedicated direct digital-to-analog converter (DAC) circuitry (349) is configured to receive the digital Pulse Density Modulation (PDM) signal and to convert it into an analog signal using a D-FlipFlop and an active lowpass filter, preferably applying a fourth order Bessel function, comprised by the dedicated direct digital-to-analog converter (DAC) circuitry (349).

12. The communication system according to any one of claims 1 - 11, wherein the one or more processors (205, 207) is further configured

- to process (603a, 619) the transmit microphone input signal (503a, 503b, 511) to generate a transmit magnitude component (621a) thereof, or
- to process (603a, 605a, 619, 625, 627) the transmit microphone input signal (503a, 503b, 511) to generate a further processed transmit magnitude component (629) thereof,

and to provide the generated transmit magnitude component (621a) or the generated further processed transmit magnitude component (629) as input to the trained artificial intelligence or machine learning method or component (515, 607) to generate the correction function or signal (517, 609).

13. The communication system according to any one of claims 1 - 12, wherein the one or more processors (205, 207) is further configured

- to process (603b, 619) the ambient microphone input signal (505a, 505b, 513) to generate an ambient magnitude component (621b) thereof, or
- to process (603a, 605b, 619, 625, 627) the ambient microphone input signal (505a, 505b, 513) to generate a further processed ambient magnitude component (633) thereof,

and to provide the generated ambient magnitude component (621b) or the generated further processed ambient magnitude component (633) as input to the trained artificial intelligence or machine learning method or component (515, 607) to generate the correction function or signal (517, 609).

14. The communication system according to claim 13, wherein the one or more processors (205, 207) is further configured

- to process (635) the generated ambient magnitude component (621b) to derive (635) a derived or estimated sound pressure value (637) thereof, the derived or estimated sound pressure value (637) representing or estimating a background noise level,

- to adjust (519, 611) the correction function or signal (517, 609), prior to the correction function or signal (517, 609) being applied to the transmit microphone input signal (503a, 503b, 511), in response to the derived or estimated sound pressure value (637) resulting in an adjusted correction function or signal (521, 649), and
- applying the adjusted correction function or signal (521, 649) to the transmit microphone input signal (503a, 503b, 511) instead of the correction function or signal (517, 609) in order to provide the corrected or improved voice signal (525, 617).

15. The communication system according to claims 12 or 13 - 14 as dependent on claim 12, wherein the one or more processors (205, 207) is further configured

- to, instead of applying the correction function or signal (517, 609) to the transmit microphone input signal (503a, 503b, 511), perform noise filtration (523, 613) of the generated further processed transmit magnitude component (629) in response to the adjusted correction function or signal (521, 649) resulting in a noise corrected further processed transmit magnitude component (653), and
- providing the corrected or improved voice signal (525, 617) in response to the noise corrected further processed transmit magnitude component (653).

16. The communication system according to any one of claims 1 - 15, wherein the communication system comprises one or more of

- a wireless remote PTT device (113),
- one or more communication devices (107, 109, 111),
- one or more radios (109, 111),
- a radio of a first type (109) and a radio of a second type (111), and
- one or more end-user-devices (ELIDs) (107).

17. The communication system according to any one of claims 1 - 16, wherein the computer program or routine implementing the trained artificial intelligence or machine learning method or component (515, 607) has been trained according to the training method (701) of any one of claims 18 - 24.

18. A method (701) of training an artificial intelligence or machine learning method or component (515, 607, 713) to be executed by at least one device (103, 303a, 303b, 107, 109, 111, 205) of a communication system, the artificial intelligence or machine learning method or component (515, 607) configured to generate real-time processing of a user's voice signal (525) in a demanding environment, the method (701) comprising

g) obtaining first data (705, 753) representing a reference speech signal including a speech signal (747) of a user (101, 101') obtained in accordance with a first way,
h) obtaining second data (707, 709, 765) representing a training transmit (Tx) signal including the speech signal (747) obtained in accordance with a second way,
i) obtaining third data (707, 711, 767) representing a training ambient signal including the speech signal (747) obtained in accordance with a third way,
j) providing the second data and the third data to the artificial intelligence or machine learning method or component (515, 607, 713) generating a predicted output (715, 717) in response thereto,
k) comparing (719) the predicted output (715, 717) and the first data (705, 753) and determining (721) a difference therebetween, and
l) updating parameters (645) of the artificial intelligence or machine learning method or component (515, 607, 713) in response to the determined difference (721, 723, 735),

wherein the method (701) further comprises repeating steps a) - f) for new first, second, and third data a plurality of times, typically a large number of times, until the generated difference of the predicted output (715, 717) and the first data (705, 753) is within a predetermined threshold or the improvement of generated difference stops improving sufficiently.

19. The method (701) of training according to claim 18, wherein

- the first way (745, 749) comprises providing an acoustic signal by a microphone or transducer of a first type being a high quality professional stationary voice recording microphone or transducer (745),
- the second way (757, 761) comprises providing an acoustic signal by a microphone or transducer of a second type being a vibration pick-up sensor (317), and/or
- the third way (759, 763) comprises providing an acoustic signal by a microphone or transducer of a third type being an ambient microphone (315).

20. The method (701) of training according to claim 18 or 19, wherein

- the second data (707, 709, 765), representing a training transmit (Tx) signal including the speech signal (747), further includes user-generated noise e.g. or preferably obtained in accordance with the second way (757, 761), and/or
- the third data (707, 711, 767), representing a training ambient signal including the speech signal (747), further includes user-generated noise e.g. or preferably obtained in accordance with the third way (759, 763).

21. The method (701) of training according to any one of claims 18 - 20, wherein

- the second data (707, 709, 765), representing a training transmit (Tx) signal including the speech signal (747), further includes noise (731, 733) being representative of loud noises of a demanding environment,
- the third data (707, 711, 767), representing a training ambient signal including the speech signal (747), further includes noise (731, 733) being representative of loud noises of a demanding environment, and
- the first data (705, 753), representing a reference speech signal including the speech signal (747), does not include noise being representative of loud noises of a demanding environment.

22. The method (701) of training according to claim 21, wherein

- the noise (731, 733) being representative of loud noises of a demanding environment of the second data (707, 709, 765) has been obtained in accordance with the second way, and/or
- the noise (731, 733) being representative of loud noises of a demanding environment of the third data (707, 711, 767) has been obtained in accordance with the third way.

23. The method (701) of training according to claim 21 or 22, wherein the noise (731, 733) being representative of loud noises of a demanding environment of the second data (707, 709, 765) and/or the noise (731, 733) being representative of loud noises of a demanding environment of the third data (707, 711, 767) have been obtained at the same time and/or have been obtained when the user (101, 101') is not speaking.

24. The method (701) of training according to any one of claims 18 - 23, wherein the method (701) further comprises

- processing (603a, 619) the second data (707, 709, 765) to generate a transmit magnitude component thereof, and providing the generated transmit magnitude component to the artificial intelligence or machine learning method or component (515, 607, 713) instead of providing the second data (707, 709, 765) in step d), and/or

- processing (603b, 619) the third data (707, 711, 767) to generate an ambient magnitude component (621b) thereof and providing the generated ambient magnitude component to the artificial intelligence or machine learning method or component (515, 607, 713) instead of providing the third data (707, 711, 767) in step d).

FIG. 1

FIG. 2A

|      | PTT-1 | PTT-2 | PTT-3 | PTT-4 |
|------|-------|-------|-------|-------|
| COM 1 | X | ( → ) | ( → ) | ( → ) |
| COM 2 |   | X | ( → ) | ( → ) |
| COM 3 |   |   | X | ( → ) |

223a · 223b · 223c · 223d · 203c · 203d · 203b · 233 · 235 · 231

## FIG. 2B

203a · 203b · 223c · 105 · 223a · 223d · 223b · 223e · 203c · 203d · 109 · 111

| INVISIO |

|      | PTT-1 | PTT-2 | PTT-3 | PTT-4 |
|------|-------|-------|-------|-------|
| COM 1 | X |   |   |   |
| COM 2 |   | X | X |   |
| COM 3 |   |   |   |   |

231

## FIG. 2C

| | PTT-1 | PTT-2 | PTT-3 | PTT-4 |
|---|---|---|---|---|
| COM 1 | X | X | | |
| COM 2 | | | X | |
| COM 3 | | | | X |

*FIG. 2D*

FIG. 3A

*FIG. 3B*

*FIG. 3C*

*FIG. 3D*

*FIG. 3E*

*FIG. 3F*

FIG. 3G

FIG. 4A

FIG. 4B

FIG. 4C

FIG. 5

FIG. 6A

*FIG. 6B*

*FIG. 6C*

FIG. 6D

*FIG. 6E*

*FIG. 6F*

*FIG. 6G*

*FIG. 7A*

FIG. 7B

FIG. 7C

FIG. 7D

Europäisches
Patentamt

European
Patent Office

Office européen
des brevets

# EUROPEAN SEARCH REPORT

**Application Number**

EP 24 22 2802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Unknown: "INVISIO V-Series Gen II", , 12 August 2024 (2024-08-12), pages 1-9, XP093278943, Internet Retrieved from the Internet: URL:https://web.archive.org/web/2024081216 3427/https://headsets.at/wp-content/upload s/Katalog-Invisio-V-Series-Gen-II-brochure -english-headsets_at.pdf [retrieved on 2025-05-19] * page 2 - page 8 * ----- | 1-16 | INV. H04R1/10 A61F11/06 G10K11/16 G10L21/00 ADD. H04R3/00 |
| A | Unknown: "INVISIO X7", , 19 April 2024 (2024-04-19), pages 1-1, XP093279209, Internet Retrieved from the Internet: URL:https://web.archive.org/web/2024041922 0743/https://invisio.com/products/headsets /invisio-x7/ [retrieved on 2025-05-19] * the whole document * ----- | 1,5,6 | |
| X | US 2019/230431 A1 (RAFT CASPER SILBO [DK]) 25 July 2019 (2019-07-25) * paragraph [0004] - paragraph [0007] * * paragraph [0038] - paragraph [0039] * * paragraph [0051] - paragraph [0063] * * paragraph [0069] * * figures 1-3 * ----- -/-- | 1-3,5,6, 9,15 | TECHNICAL FIELDS SEARCHED (IPC) H04R G10L G10K A61F |

The present search report has been drawn up for all claims

| Place of search | Date of completion of the search | Examiner |
|---|---|---|
| Munich | 9 July 2025 | Meiser, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 24 22 2802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Unknown: "INVISIO X7 - Redefining the usability of tactical in-ear headsets", , 19 April 2024 (2024-04-19), pages 1-8, XP093279372, Internet Retrieved from the Internet: URL:https://web.archive.org/web/2024041922 0743/https://invisio.com/products/headsets /invisio-x7/ [retrieved on 2025-05-19] * page 4 - page 6 * | 4,6 | |
| X | Mcdowell Tiffany: "How AI voice works and why it's important", , 13 June 2024 (2024-06-13), pages 1-7, XP093293927, Retrieved from the Internet: URL:https://telnyx.com/resources/how-ai-vo ice-works [retrieved on 2025-07-08] | 17,18 | |
| Y | *page 3, "Core components of AI voice systems" as well as "Automatic speech recognition (ASR)"* *page 4, steps "Data collection" - "Continuous improvement"* | 19-24 | TECHNICAL FIELDS SEARCHED (IPC) |

-/--

The present search report has been drawn up for all claims

| Place of search | Date of completion of the search | Examiner |
|---|---|---|
| Munich | 9 July 2025 | Meiser, Jürgen |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................................
& : member of the same patent family, corresponding
    document

EPO FORM 1503 03.82 (P04C01)

page 2 of 3

Europäisches
Patentamt

European
Patent Office

Office européen
des brevets

**EUROPEAN SEARCH REPORT**

Application Number

EP 24 22 2802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous Daniel: "Learn to Train AI Voice [Step-by-Step Training Manual]", , 10 April 2024 (2024-04-10), pages 1-12, XP093293930, Retrieved from the Internet: URL:https://www.topmediai.com/text-speaker /train-ai-voice/ [retrieved on 2025-07-08] | 17,18 | |
| Y | *page3, steps "Preprocessing" – "Monitoring and Updates"* ----- | 19-24 | |
| Y | US 2019/385614 A1 (KIM JAEHONG [KR] ET AL) 19 December 2019 (2019-12-19) * paragraph [0054] – paragraph [0060] * * paragraph [0201] – paragraph [0214] * * figure 1 * ----- | 19-24 | |

TECHNICAL FIELDS
SEARCHED (IPC)

The present search report has been drawn up for all claims

| Place of search | Date of completion of the search | Examiner |
|---|---|---|
| Munich | 9 July 2025 | Meiser, Jürgen |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

The Search Division considers that the present European patent application does not comply with the requirements of unity of invention and relates to several inventions or groups of inventions, namely:

1. claims: 1-16

   A communication system configured to be used in a demanding environment, the communication system comprising- at least one communication and hearing protection device, in particular at least one in-ear communication and hearing protection device, configured to be worn by a user, the communication and hearing protection device comprising an ambient microphone configured to provide an ambient microphone input signal, and a transmit microphone configured to provide a transmit microphone input signal in response to registering speech from the user,- one or more processors configured to implement and execute a trained artificial intelligence or machine learning method or component to generate a correction function or signal in response to the ambient microphone input signal and the transmit microphone input signal.

   ---

2. claims: 17-24

   A method of training an artificial intelligence or machine learning method or component to be executed by at least one device of a communication system, the artificial intelligence or machine learning method or component configured to generate real-time processing of a user's voice signal in a demanding environment, the method comprising g) obtaining first data representing a reference speech signal including a speech signal of a user obtained in accordance with a first way, h) obtaining second data representing a training transmit signal including the speech signal obtained in accordance with a second way, i) obtaining third data representing a training ambient signal including the speech signal obtained in accordance with a third way, i) providing the second data and the third data to the artificial intelligence or machine learning method or component generating a predicted output in response thereto, k) comparing the predicted output and the first data and determining a difference therebetween, and l) updating parameters of the artificial intelligence or machine learning method or component in response to the determined difference,wherein the method further comprises repeating steps a) - f) for new first, second, and third data a plurality of times, typically a large number of times, until the generated difference of the predicted output and the first data is within a predetermined threshold or the improvement of generated difference stops improving sufficiently.

   ---

## ANNEX TO THE EUROPEAN SEARCH REPORT
## ON EUROPEAN PATENT APPLICATION NO.

EP 24 22 2802

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2025

| Patent document cited in search report | Publication date | Patent family member(s) | | Publication date |
|---|---|---|---|---|
| US 2019230431 A1 | 25-07-2019 | AU | 2019200428 A1 | 08-08-2019 |
| | | CN | 110072168 A | 30-07-2019 |
| | | DK | 3517083 T3 | 07-02-2022 |
| | | EP | 3517083 A1 | 31-07-2019 |
| | | IL | 264275 A | 30-05-2019 |
| | | US | 2019230431 A1 | 25-07-2019 |
| | | US | 2022046353 A1 | 10-02-2022 |
| US 2019385614 A1 | 19-12-2019 | KR | 20190104490 A | 10-09-2019 |
| | | US | 2019385614 A1 | 19-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2845115 B1
- EP 3298800 B1
- EP 24174205
- EP 24182433
- EP 24189349